# EUROPEAN PATENT APPLICATION

(11) **EP 4 502 158 A1**
(43) Date of publication of application: **05.02.2025**
(21) Application number: 23773667.3
(22) Date of filing: 15.03.2023
(51) Int. Cl.: C12N 15/113, C12N 15/861, C12N 15/86, A61K 48/00, A61K 38/17

(54) **COCHLEAR AND/OR VESTIBULAR CELL-SPECIFIC PROMOTER AND USE THEREOF**

(30) Priority: 25.03.2022 CN 202210321731
(71) Applicant: Shanghai Emaygene Technology Co., Ltd., Shanghai 201203 (CN)
(72) Inventor: ZHONG, Guisheng, Shanghai 201203 (CN); CHU, Cenfeng, Shanghai 201203 (CN)
(74) Representative: Canzler & Bergmeier Patentanwälte Partnerschaft mbB
(86) International application number: PCT/CN2023/081566
(87) International publication number: WO 2023/179419

(57) **Abstract**

The present invention provides a cochlear and/or vestibular cell-specific promoter and use thereof, and specifically relates to specific promoters STRC, Slc26a5, otof, Sox10, Ngfr1, Ngfr2, and Dnah5 and use thereof in cochlear and/or vestibular cells. The nucleotide sequences of the specific promoters are as set forth in SEQ ID NOs. 1, 6, 11, 16, 21, 26, and 31, respectively. Also provided is a recombinant vector comprising the specific promoters. On the basis of the fact that the promoters are characterized by specific expression in cochlea and/or vestibule, use of the tissue-specific promoters, an amplification primer, the recombinant vector, or a pharmaceutical composition in gene therapies, construction of related animal models, pathogenesis-related researches, and the like for hearing-related diseases is provided.

## Description

### FIELD OF TECHNOLOGY

The present disclosure belongs to the field of biomedicine, and relates to a cochlear and/or vestibular cell-specific promoter and use thereof, and specifically to specific promoters STRC, Slc26a5, otof, Sox10, Ngfr 1, Ngfr 2, Dnah5 and use thereof in cochlear and/or vestibular cells.

### BACKGROUND

Hearing loss is one of the most common sensorineural diseases in the world. According to statistics from the World Health Organization, as of March 2020, approximately 466 million people worldwide have hearing impairments, accounting for 5% of the global population, among which 34 million are children. Among every 1,000 newborns, there are three with congenital hearing loss. Genetic factors are considered to be predisposing factors or immediate causes for 50-60% of sensorineural hearing loss. The primary functional cells in cochlea are categorized as hair cells, supporting cells and spiral neurons, etc., in which deafness-causing gene is distributed, therefore, specific regulation of gene expression is of great significance for hearing gene therapies.

Currently, researchers primarily rely on transgenic animals for functional analysis of specific cell types. However, transgenic animals such as mice, rats, dogs, pigs, and monkeys have long reproduction cycles and high maintenance costs, and cannot flexibly carry target genes for the manipulation of cells and treatment of diseases. Therefore, it is of great significance to further develop methods that can be used for labeling and manipulating specific cell types. With the development of bioinformatics and molecular biology, the use of specific promoters to mediate the expression of exogenous genes has become an efficient way for targeting and manipulating specific tissues, as well as for gene therapies.

Deafness-causing genes are mostly distributed in hair cells. Therefore, specific regulation of gene expression in hair cells is of great significance for hearing gene therapies. Currently, no promoters capable of specifically expressing exogenous genes have been identified in hair cells of the auditory system. The promoter used for gene therapies is the constitutive promoter CAG or CMV, and therefore exogenous genes can be expressed in cells other than hair cells, causing side effects of gene therapies. Additionally, when exogenous genes are introduced by means of virus injection, e.g., when viruses are injected into young mice through a round window, the viruses will enter the brain tissue through the cerebrospinal fluid, thereby infecting cells in the brain tissue. Therefore, there is an urgent need to develop a method that can reduce the expression of exogenous genes in cells other than hair cells, such as brain tissue cells, so as to improve the safety of gene therapies.

Hair cells are further categorized as outer hair cells and inner hair cells, the inner hair cells are responsible for analyzing the sound information, while the outer hair cells are responsible for amplifying the sound. There are many specifically-expressed proteins in outer hair cells, which have great significance to the function of outer hair cells. Some deafness-causing genes are specifically distributed in inner hair cells or outer hair cells, therefore, more specific regulation is of great significance for precision treatment. Prestin is a highly specialized anionic carrier, belonging to the superfamily of solute carriers SLC26 (solute carrier protein), and is specifically distributed on side walls of outer hair cells (OHCs) of organ of Corti in the inner ear, which is the molecular basis of electrokinetic movement and auditory amplification of outer hair cells and is also known as motor protein. Likewise, prestin is also a kind of deafness-causing gene. Therefore, specific regulation of gene expression in outer hair cells is of great significance for the functional research of outer hair cells and hearing gene therapies.

Currently, no promoters capable of specifically expressing exogenous genes have been identified in outer hair cells of the auditory system. The promoter used for gene therapies is the constitutive promoter CAG or CMV, and therefore exogenous genes can be expressed in cells other than outer hair cells, causing side effects of gene therapies. Some deafness-causing genes are specifically distributed in inner hair cells or outer hair cells, so more specific regulation has great significance to precision treatment. Additionally, when viruses are injected into young mice through a round window, the viruses will enter the brain tissue through the cerebrospinal fluid, thereby infecting cells in the brain tissue. Therefore, there is an urgent need to develop a method that can reduce the expression of exogenous genes in cells other than outer hair cells, such as brain tissue cells, so as to improve the safety of gene therapies.

Hair cells are further categorized as inner hair cells and outer hair cells, the outer hair cells are responsible for amplifying the sound, while the inner hair cells are responsible for analyzing the sound information. Some deafness-causing genes such as otof, vglut3, etc. are mainly distributed in inner hair cells, therefore, specific regulation of gene expression in inner hair cells is of great significance for the functional research of inner hair cells and hearing gene therapies. Currently, no promoters capable of specifically expressing exogenous genes have been identified in inner hair cells of the auditory system. The promoter used for gene therapies is the constitutive promoter CAG or CMV, and therefore exogenous genes can be expressed in cells other than inner hair cells, causing side effects of gene therapies. Some deafness-causing genes are specifically distributed in inner hair cells, therefore, more specific regulation is of great significance for precision treatment. Additionally, when viruses are injected into young mice through a round window, the viruses will enter the brain tissue through the cerebrospinal fluid, thereby infecting cells in the brain tissue. Therefore, there is an urgent need to develop a method that can reduce the expression of exogenous genes in cells other than inner hair cells, such as brain tissue cells, so as to improve the safety of gene therapies.

Deafness-causing genes are mostly distributed in hair cells. The most common deafness-causing gene in China, Gjb2, is mainly expressed in supporting cells, accounting for more than 30% of congenital deafness cases. In addition, the transformation of supporting cells into hair cells is considered as an important pathway of hair cell regeneration. Therefore, specific regulation of gene expression in supporting cells is of great significance for the functional research of supporting cells, hearing gene therapies and hair cell regeneration.

Currently, no promoters capable of specifically expressing exogenous genes have been identified in supporting cells of the auditory system. The promoter used for gene therapies is the constitutive promoter CAG or CMV, and therefore exogenous genes can be expressed in cells other than supporting cells, causing side effects of gene therapies. Some deafness-causing genes are specifically distributed in hair cells or supporting cells, therefore, more specific regulation is of great significance for precision treatment. Additionally, when viruses are injected into young mice through a round window, the viruses will enter the brain tissue through the cerebrospinal fluid, thereby infecting cells in the brain tissue. Therefore, there is an urgent need to develop a method that can reduce the expression of exogenous genes in cells other than supporting cells, such as brain tissue cells, so as to improve the safety of gene therapies.

Spiral ganglion neurons (SGNs) are primary afferent neurons of the auditory system, which transmit sound information from hair cells to the cochlear nucleus of the brainstem to produce hearing. Therefore, specific regulation of gene expression in spiral ganglion neurons is of great significance for the functional research of spiral ganglion neurons and hearing gene therapies. Spiral ganglion neurons are divided into type I spiral ganglion neurons and type II spiral ganglion neurons, type I spiral ganglion neurons are connected with inner hair cells, accounting for 95% of all the spiral ganglion neurons and participating in the transmission of sound information analysis; and type II spiral ganglion neurons are connected with outer hair cells, accounting for 5% of all the spiral ganglion neurons and being involved in the amplification of sound. However, the structure and function of type I or type II spiral ganglion neurons have not yet been clearly studied.

Currently, no promoters capable of specifically expressing exogenous genes have been identified in type I spiral ganglion neurons of the auditory system. The promoter used for gene therapies is the constitutive promoter CAG or CMV, and therefore exogenous genes can be expressed in cells other than target cells, causing side effects of gene therapies. Some deafness-causing genes are specifically distributed in type I or type II spiral ganglion neurons, therefore, more specific regulation is of great significance for precision treatment. Additionally, when viruses are injected into young mice through a round window, the viruses will enter the brain tissue through the cerebrospinal fluid, thereby infecting cells in the brain tissue. Therefore, there is an urgent need to develop a method that can reduce the expression of exogenous genes in cells other than spiral ganglion neurons, such as brain tissue cells, so as to improve the safety of gene therapies.

Vertiginous diseases are a group of common and frequently occurring conditions, with a prevalence rate as high as 4.9% in the population, and their causes are diverse and complex, involving a number of disciplines. The vestibular system is an important part of the human balance system, for which the receptors are otolith organs and semicircular canals located in the inner ear. The receptors receive appropriate stimulatory signals, which are then transmitted via the vestibular nerve to the vestibular nuclei, where the stimulatory signals are integrated with other sensory signals to perform specific or non-specific functions via multiple conduction pathways. Vestibular hair cells are mechanosensory receptors that can detect changes in head position, enabling animals to maintain posture and coordinate movement. The function of vestibular hair cells is to convert mechanical motion signals into bioelectrical signals. Vestibular dysfunction is primarily caused by damage to hair cells located in the vestibular sensory epithelium. Vestibular hair cells are susceptible to ototoxic drugs, aging and genetic factors, that may lead to permanent vestibular dysfunction. Gene mutations can lead to the loss of vestibular hair cells. For instance, the Myo15a:whirlin:Esp8 complex at the tip of cilia is crucial for the growth of cilia, and mutations in genes related to cilia function can result in damage to vestibular hair cells. Therefore, specific regulation of gene expression in vestibular hair cells is of great significance for the functional research of vestibular hair cells, hearing gene therapies and vestibular hair cell regeneration.

Currently, no promoters capable of specifically expressing exogenous genes have been identified in vestibular hair cells. The promoter used for gene therapies is the constitutive promoter CAG or CMV, and therefore exogenous genes can be expressed in cells other than hair cells, causing side effects of gene therapies. Some vestibule-related disease genes are specifically distributed in vestibular hair cells or supporting cells, therefore, more specific regulation is of great significance for precision treatment. Additionally, when viruses are injected into young mice through a round window, the viruses will enter the brain tissue through the cerebrospinal fluid, thereby infecting cells in the brain tissue. While with our promoters, the expression of exogenous genes in brain tissues can be reduced, thereby improving the safety of gene therapies. Therefore, there is an urgent need to develop a method that can reduce the expression of exogenous genes in cells other than vestibular hair cells, such as brain tissue cells, so as to improve the safety of gene therapies.

### SUMMARY

### I. Cochlear and/or vestibular hair cell-specific promoter STRC

The present disclosure provides a cochlear and/or vestibular hair cell-specific promoter and use thereof. The promoter is a hair cell-specific promoter obtained from the genome of C57BL/6J strain mice, which can regulate the specific expression of genes in the hair cells. In the present disclosure, the promoter is loaded into an adeno-associated viral vector to infect the cochlea and vestibule of mice in vivo, which can mediate the target gene to be specifically expressed in the cochlear and vestibular hair cells, thereby labeling or manipulating the hair cells. Such a promoter has extensive application value and market prospects in terms of the structural and functional analysis of cochlear and vestibular hair cells, auditory neuroscience, modeling of deafness diseases, gene therapies and the like.

The present disclosure provides a cochlear and/or vestibular hair cell-specific promoter,
a) the specific promoter comprises a nucleotide sequence shown as SEQ ID NO: 1; or
b) the specific promoter comprises a nucleotide fragment having a sequence identity of more than 80% to SEQ ID NO: 1 and having a function of the specific promoter defined in a).

In the present disclosure, the nucleotide fragment in b) specifically refers to: a nucleotide fragment obtained from substitution, deletion or addition of one or more (specifically, 1-50, 1-30, 1-20, 1-10, 1-5, 1-3, 1, 2, or 3) nucleotides to the nucleotide sequence shown as SEQ ID NO: 1, or from addition of one or more (specifically, 1-50, 1-30, 1-20, 1-10, 1-5, 1-3, 1, 2, or 3) nucleotides at its 5'-end and/or 3'-end, and having the function of the nucleotide fragment with a sequence shown as SEQ ID NO: 1. The nucleotide sequence in b) may have a sequence identity of 80%, 81%, 82%, 83%, 84%, 85%, 86%, 87%, 88%, 89%, 90%, 91%, 92%, 93%, 94%, 95%, 96%, 97%, 98% or 99% or more to SEQ ID NO: 1.

The promoter of the present disclosure comprises a nucleic acid having a sequence identity of at least 80%, preferably having a sequence identity of at least 90%, preferably having a sequence identity of at least 95%, further preferably having a sequence identity of at least 98% to the nucleotide sequence shown as SEQ ID NO: 1; more further preferably, the promoter comprises the nucleotide sequence shown as SEQ ID NO: 1; particularly preferably, the promoter is the nucleotide sequence shown as SEQ ID NO: 1. The promoter may comprise, in addition to nucleotides having a sequence identity of at least 80% to the nucleotide sequence shown as SEQ ID NO: 1, base sequences at its terminals, such as restriction enzyme sites, att sequences for Gateway cloning and other sites for cloning into vectors.

In some embodiments, the specific promoter is STRC, with its nucleotide sequence shown as SEQ ID NO: 1. In some embodiments, the specific promoter is a shortened promoter STRC mini, with its nucleotide sequence shown as SEQ ID NO: 2.

The present disclosure provides a primer pair A for amplifying the above specific promoter, comprising a forward primer and a reverse primer, the nucleotide sequence of the forward primer is shown as SEQ ID NO: 3; and the nucleotide sequence of the reverse primer is shown as SEQ ID NO: 4.

The present disclosure provides a cochlear and/or vestibular hair cell-specific recombinant vector, comprising the above specific promoter.

In some embodiments, the gene of the specific promoter STRC is inserted at a Kpn1/Mlu1 or Xbal/BamHI multiple cloning site of a basic vector. Preferably, the specific promoter STRC is inserted at a Kpn1/Mlu1 multiple cloning site of a basic vector.

In some embodiments, the recombinant vector further comprises a target gene. In some embodiments, the target gene is located at the 3' end of the gene of the specific promoter. In some embodiments, the target gene is expressed in the cochlear and/or vestibular cell. In some embodiments, the target gene is selected from one or more of human deafness-causing genes, such as Prestin, otof, vglut3, and Gjb2.

The present disclosure provides a pharmaceutical composition, comprising the above recombinant vector.

The present disclosure provides a use of the specific promoter, the primer pair, the recombinant vector or the pharmaceutical composition, which is at least any one selected from:
1) use in preparing a medicine for preventing hearing-related diseases;
2) use in preparing a medicine for gene therapies of hearing-related diseases;
3) use in preparing and screening a medicine or vector for preventing and/or treating hearing-related diseases;
4) use in constructing animal models of hearing-related diseases;
5) use in constructing animal models of diseases accompanied with deafness genetic abnormalities;
6) use in in vitro studies related to cochlear and/or vestibular hair cell regeneration;
7) use in in vitro studies related to cochlear and/or vestibular hair cell damage protection;
8) use in in vitro studies related to inner ear development;
9) use in in vitro studies related to the pathogenesis of hearing-related diseases;
10) use in in vitro studies related to auditory synaptic reconstruction.

The present disclosure provides a method for treating hearing-related diseases, comprising: administering to a patient in need thereof an effective amount of the recombinant vector.

In one embodiment, the present disclosure provides a use of the cochlea-specific promoter STRC or the shortened promoter STRC mini to specifically initiate the expression of an exogenous gene in cochlear and/or vestibular hair cells, in which the promoter STRC is loaded into an adeno-associated viral vector and infect the cochlear and vestibular hair cells of mice in vivo, with the results indicating that the STRC promoter is capable of specifically initiating the expression of mNeonGreen gene (a kind of fluorescent protein) in cochlear and vestibular hair cells.

In one embodiment, the present disclosure provides a use of the specific promoter STRC in constructing transgenic mouse models, in which the promoter STRC or the shortened promoter STRC mini is loaded into an adeno-associated viral vector, and at the same time, the target gene is constructed onto the adeno-associated viral vector, so that the transcription and expression of the target gene can be controlled so as to construct transgenic mouse models.

The promoter STRC or the shortened promoter STRC mini of the present disclosure is the promoter which specifically acts in cochlear and vestibular hair cells, thereby enabling the gene operatively attached at the downstream of the promoter to be specifically expressed in the cochlear and vestibular hair cells. The promoter of the present disclosure can be obtained by gene engineering. For example, the gene of the human promoter STRC is obtained from human genome by deletion, insertion or replacement of partial bases to get promoters having desired base sequences. Additionally, the promoters having desired base sequences can also be obtained by partial synthesis or total synthesis.

Compared with the prior art, the specific promoter STRC or the shortened promoter STRC mini of the present disclosure has the following advantages and beneficial effects:
The present disclosure provides a hair cell-specific promoter STRC or a shortened promoter STRC mini, which can mediate the target gene to be specifically expressed in the cochlear and/or vestibular hair cells of mice, thereby labeling or manipulating the hair cells.

The specific promoter STRC or the shortened promoter STRC mini of the present disclosure can reduce the expression of exogenous genes in other tissues, e.g., brain tissues, thereby improving the safety of gene therapies.

The specific promoter STRC or the shortened promoter STRC mini of the present disclosure mediates the expression of the target gene, which is more flexible, more convenient in application and lower in cost than the traditional transgenic methods.

The specific promoter STRC or the shortened promoter STRC mini of the present disclosure can be loaded into desired vectors, and at the same time, it can express therapeutic genes for gene therapies, which cannot be achieved with transgenic animals.

The specific promoter STRC or the shortened promoter STRC mini of the present disclosure has extensive application value and market prospects in auditory neuroscience, modeling of deafness diseases, gene therapies and other fields.

### II. Cochlear outer hair cell-specific promoter Slc26a5

The present disclosure provides a cochlear outer hair cell-specific promoter and use thereof. The promoter is an outer hair cell-specific promoter obtained from the genome of C57BL/6J strain mice, which can regulate the specific expression of genes in the outer hair cells. In the present disclosure, the promoter is loaded into an adeno-associated viral vector to infect the cochlea of mice in vivo, which can mediate the target gene to be specifically expressed in the cochlear outer hair cells, thereby labeling or manipulating the outer hair cells. Such a promoter has extensive application value and market prospects in terms of the structural and functional analysis of cochlear outer hair cells, auditory neuroscience, modeling of deafness diseases, gene therapies and the like.

One of the objectives of the present disclosure is to provide a cochlear outer hair cell-specific promoter,
a) the specific promoter comprises a nucleotide sequence shown as SEQ ID NO: 6; or
b) the specific promoter comprises a nucleotide fragment having a sequence identity of more than 80% to SEQ ID NO: 6 and having a function of the specific promoter defined in a).

In the present disclosure, the nucleotide fragment in b) specifically refers to: a nucleotide fragment obtained from substitution, deletion or addition of one or more (specifically, 1-50, 1-30, 1-20, 1-10, 1-5, 1-3, 1, 2, or 3) nucleotides to the nucleotide sequence shown as SEQ ID NO: 6, or from addition of one or more (specifically, 1-50, 1-30, 1-20, 1-10, 1-5, 1-3, 1, 2, or 3) nucleotides at its 5'-end and/or 3'-end, and having the function of the nucleotide fragment with a sequence shown as SEQ ID NO: 6. The nucleotide sequence in b) may have a sequence identity of 80%, 81%, 82%, 83%, 84%, 85%, 86%, 87%, 88%, 89%, 90%, 91%, 92%, 93%, 94%, 95%, 96%, 97%, 98% or 99% or more to SEQ ID NO: 6.

The promoter of the present disclosure comprises a nucleic acid having a sequence identity of at least 80%, preferably having a sequence identity of at least 90%, preferably having a sequence identity of at least 95%, further preferably having a sequence identity of at least 98% to the nucleotide sequence shown as SEQ ID NO: 6; more further preferably, the promoter comprises the nucleotide sequence shown as SEQ ID NO: 6; particularly preferably, the promoter is the nucleotide sequence shown as SEQ ID NO: 6. The promoter may comprise, in addition to nucleotides having a sequence identity of at least 80% to the nucleotide sequence shown as SEQ ID NO: 6, base sequences at its terminals, such as restriction enzyme sites, att sequences for Gateway cloning and other sites for cloning into vectors.

In some embodiments, the specific promoter is Slc26a5, with its nucleotide sequence shown as SEQ ID NO: 6.

The present disclosure provides a primer pair B for amplifying the above specific promoter, comprising a forward primer and a reverse primer, the nucleotide sequence of the forward primer is shown as SEQ ID NO: 8; and the nucleotide sequence of the reverse primer is shown as SEQ ID NO: 9.

The present disclosure provides a cochlear outer hair cell-specific recombinant vector, comprising the above specific promoter.

In some embodiments, the gene of the specific promoter Slc26a5 is inserted at a Kpn1/Mlu1 or Xbal/BamHI multiple cloning site of a basic vector. Preferably, the specific promoter Slc26a5 is inserted at the Xbal/BamHI multiple cloning site of the basic vector.

In some embodiments, the recombinant vector further comprises a target gene. In some embodiments, the target gene is located at the 3' end of the gene of the specific promoter. In some embodiments, the target gene is expressed in the cochlear outer hair cells. In some embodiments, the target gene is selected from one or more of human deafness-causing genes, such as Prestin, otof, vglut3, and Gjb2.

The present disclosure provides a pharmaceutical composition, comprising the above recombinant vector.

The present disclosure provides a use of the specific promoter, the primer pair, the recombinant vector or the pharmaceutical composition, which is at least any one selected from:
1) use in preparing a medicine for preventing hearing-related diseases;
2) use in preparing a medicine for gene therapies of hearing-related diseases;
3) use in preparing and screening a medicine or vector for preventing and/or treating hearing-related diseases;
4) use in constructing animal models of hearing-related diseases;
5) use in constructing animal models of diseases accompanied with deafness genetic abnormalities;
6) use in in vitro studies related to cochlear outer hair cell regeneration;
7) use in in vitro studies related to cochlear outer hair cell damage protection;
8) use in in vitro studies related to inner ear development;
9) use in in vitro studies related to the pathogenesis of hearing-related diseases;
10)use in in vitro studies related to auditory synaptic reconstruction.

The present disclosure provides a method for treating hearing-related diseases, comprising: administering to a patient in need thereof an effective amount of the recombinant vector.

In one embodiment, the present disclosure provides a use of the cochlea-specific promoter Slc26a5 to specifically initiate the expression of an exogenous gene in cochlear outer hair cells, in which the promoter Slc26a5 is loaded into an adeno-associated viral vector and infect the cochlea of mice in vivo, with the results indicating that promoter Slc26a5 is capable of specifically initiating the expression of mNeonGreen gene (a kind of fluorescent protein) in cochlear outer hair cells.

In one embodiment, the present disclosure provides a use of the specific promoter Slc26a5 in constructing transgenic mouse models, in which the promoter Slc26a5 is loaded into an adeno-associated viral vector, and at the same time, the target gene is constructed onto the adeno-associated viral vector, so that the transcription and expression of the target gene can be controlled so as to construct transgenic mouse models.

The promoter Slc26a5 of the present disclosure is the promoter which specifically acts in cochlear outer hair cells, thereby enabling the gene operatively attached at the downstream of the promoter to be specifically expressed in the cochlear outer hair cells. The promoter Slc26a5 of the present disclosure can be obtained by gene engineering. For example, the gene of the human promoter Slc26a5 is obtained from human genome by deletion, insertion or replacement of partial bases to get promoters having desired base sequences. Additionally, the promoters having desired base sequences can also be obtained by partial synthesis or total synthesis.

Compared with the prior art, the specific promoter Slc26a5 of the present disclosure has the following advantages and beneficial effects:
The present disclosure provides a hair cell-specific promoter Slc26a5, which can mediate the target gene to be specifically expressed in the cochlear outer hair cells of mice, thereby labeling or manipulating the cochlear outer hair cells.

The specific promoter Slc26a5 of the present disclosure can reduce the expression of exogenous genes in other tissues, e.g., brain tissues, thereby improving the safety of gene therapies.

The specific promoter Slc26a5 of the present disclosure mediates the expression of the target gene, which is more flexible, more convenient in application and lower in cost than the traditional transgenic methods.

The specific promoter Slc26a5 of the present disclosure can be loaded into desired vectors, and at the same time, it can express therapeutic genes for gene therapies, which cannot be achieved with transgenic animals.

The specific promoter Slc26a5 of the present disclosure has extensive application value and market prospects in auditory neuroscience, modeling of deafness diseases, gene therapies and other fields.

### III Cochlear inner hair cell-specific promoter otof

The present disclosure provides a cochlear inner hair cell-specific promoter and use thereof. The promoter is an inner hair cell-specific promoter obtained from the genome of C57BL/6J strain mice, which can regulate the specific expression of genes in the inner hair cells. In the present disclosure, the promoter is loaded into an adeno-associated viral vector to infect the cochlea of mice in vivo, which can mediate the target gene to be specifically expressed in the cochlear inner hair cells, thereby labeling or manipulating the inner hair cells. Such a promoter has extensive application value and market prospects in terms of the structural and functional analysis of cochlear inner hair cells, auditory neuroscience, modeling of deafness diseases, gene therapies and the like.

One of the objectives of the present disclosure is to provide a cochlear inner hair cell-specific promoter,
a) the specific promoter comprises a nucleotide sequence shown as SEQ ID NO: 11; or
b) the specific promoter comprises a nucleotide fragment having a sequence identity of more than 80% to SEQ ID NO: 11 and having a function of the specific promoter defined in a).

In the present disclosure, the nucleotide fragment in b) specifically refers to: a nucleotide fragment obtained from substitution, deletion or addition of one or more (specifically, 1-50, 1-30, 1-20, 1-10, 1-5, 1-3, 1, 2, or 3) nucleotides to the nucleotide sequence shown as SEQ ID NO: 11, or from addition of one or more (specifically, 1-50, 1-30, 1-20, 1-10, 1-5, 1-3, 1, 2, or 3) nucleotides at its 5'-end and/or 3'-end, and having the function of the nucleotide fragment with a sequence shown as SEQ ID NO: 11. The nucleotide sequence in b) may have a sequence identity of 80%, 81%, 82%, 83%, 84%, 85%, 86%, 87%, 88%, 89%, 90%, 91%, 92%, 93%, 94%, 95%, 96%, 97%, 98% or 99% or more to SEQ ID NO: 11.

The promoter of the present disclosure comprises a nucleic acid having a sequence identity of at least 80%, preferably having a sequence identity of at least 90%, preferably having a sequence identity of at least 95%, further preferably having a sequence identity of at least 98% to the nucleotide sequence shown as SEQ ID NO: 11; more further preferably, the promoter comprises the nucleotide sequence shown as SEQ ID NO: 11; particularly preferably, the promoter is the nucleotide sequence shown as SEQ ID NO: 11. The promoter may comprise, in addition to nucleotides having a sequence identity of at least 80% to the nucleotide sequence shown as SEQ ID NO: 11, base sequences at its terminals, such as restriction enzyme sites, att sequences for Gateway cloning and other sites for cloning into vectors.

In some embodiments, the specific promoter is otof, with its nucleotide sequence shown as SEQ ID NO: 11.

The present disclosure provides a primer pair C for amplifying the above specific promoter, comprising a forward primer and a reverse primer, the nucleotide sequence of the forward primer is shown as SEQ ID NO: 13; and the nucleotide sequence of the reverse primer is shown as SEQ ID NO: 14.

The present disclosure provides a cochlear inner hair cell-specific recombinant vector, comprising the above specific promoter.

In some embodiments, the gene of the specific promoter otof is inserted at a Kpn1/Mlu1 or Xbal/BamHI multiple cloning site of a basic vector. Preferably, the specific promoter otof is inserted at the Xbal/BamHI multiple cloning site of the basic vector.

In some embodiments, the recombinant vector further comprises a target gene. In some embodiments, the target gene is located at the 3' end of the gene of the specific promoter. In some embodiments, the target gene is expressed in the cochlear inner hair cells. In some embodiments, the target gene is selected from one or more of human deafness-causing genes, such as Prestin, otof, vglut3, and Gjb2.

The present disclosure provides a pharmaceutical composition, comprising the above recombinant vector.

The present disclosure provides a use of the specific promoter, the primer pair, the recombinant vector or the pharmaceutical composition, which is at least any one selected from:
1) use in preparing a medicine for preventing hearing-related diseases;
2) use in preparing a medicine for gene therapies of hearing-related diseases;
3) use in preparing and screening a medicine or vector for preventing and/or treating hearing-related diseases;
4) use in constructing animal models of hearing-related diseases;
5) use in constructing animal models of diseases accompanied with deafness genetic abnormalities;
6) use in in vitro studies related to cochlear inner hair cell regeneration;
7) use in in vitro studies related to cochlear inner hair cell damage protection;
8) use in in vitro studies related to inner ear development;
9) use in in vitro studies related to the pathogenesis of hearing-related diseases;
10) use in in vitro studies related to auditory synaptic reconstruction.

The present disclosure provides a method for treating hearing-related diseases, comprising: administering to a patient in need thereof an effective amount of the recombinant vector.

In one embodiment, the present disclosure provides a use of the cochlea-specific promoter otof to specifically initiate the expression of an exogenous gene in cochlear inner hair cells, in which the promoter otof is loaded into an adeno-associated viral vector and infect the cochlea of mice in vivo, with the results indicating that promoter otof is capable of specifically initiating the expression of mNeonGreen gene (a kind of fluorescent protein) in cochlear inner hair cells.

In one embodiment, the present disclosure provides a use of the specific promoter otof in constructing transgenic mouse models, in which the promoter otof is loaded into an adeno-associated viral vector, and at the same time, the target gene is constructed onto the adeno-associated viral vector, so that the transcription and expression of the target gene can be controlled so as to construct transgenic mouse models.

The promoter otof of the present disclosure is the promoter which specifically acts in cochlear inner hair cells, thereby enabling the gene operatively attached at the downstream of the promoter to be specifically expressed in the cochlear inner hair cells. The promoter otof of the present disclosure can be obtained by gene engineering. For example, the gene of the human promoter otof is obtained from human genome by deletion, insertion or replacement of partial bases to get promoters having desired base sequences. Additionally, the promoters having desired base sequences can also be obtained by partial synthesis or total synthesis.

Compared with the prior art, the specific promoter otof of the present disclosure has the following advantages and beneficial effects:
The present disclosure provides a hair cell-specific promoter otof, which can mediate the target gene to be specifically expressed in the cochlear inner hair cells of mice, thereby labeling or manipulating the inner hair cells.

The specific promoter otof of the present disclosure can reduce the expression of exogenous genes in other tissues, e.g., brain tissues, thereby improving the safety of gene therapies.

The specific promoter otof of the present disclosure mediates the expression of the target gene, which is more flexible, more convenient in application and lower in cost than the traditional transgenic methods.

The specific promoter otof of the present disclosure can be loaded into desired vectors, and at the same time, it can express therapeutic genes for gene therapies, which cannot be achieved with transgenic animals.

The specific promoter otof of the present disclosure has extensive application value and market prospects in auditory neuroscience, modeling of deafness diseases, gene therapies and other fields.

### IV. Cochlear supporting cell-specific promoter

The present disclosure provides a cochlear supporting cell-specific promoter and use thereof. The promoter is a supporting cell-specific promoter obtained from the genome of C57BL/6J strain mice, which can regulate the specific expression of genes in the supporting cells. In the present disclosure, the promoter is loaded into an adeno-associated viral vector to infect the cochlea of mice in vivo, which can mediate the target gene to be specifically expressed in the cochlear supporting cells, thereby labeling or manipulating the supporting cells. Such a promoter has extensive application value and market prospects in terms of the structural and functional analysis of cochlear supporting cells, auditory neuroscience, modeling of diseases, gene therapies and the like.

One of the objectives of the present disclosure is to provide a cochlear supporting cell-specific promoter, comprising:
a) the specific promoter comprises a nucleotide sequence shown as SEQ ID NO: 16; or
b) the specific promoter comprises a nucleotide fragment having a sequence identity of more than 80% to SEQ ID NO: 16 and having a function of the specific promoter defined in a).

In the present disclosure, the nucleotide fragment in b) specifically refers to: a nucleotide fragment obtained from substitution, deletion or addition of one or more (specifically, 1-50, 1-30, 1-20, 1-10, 1-5, 1-3, 1, 2, or 3) nucleotides to the nucleotide sequence shown as SEQ ID NO: 16, or from addition of one or more (specifically, 1-50, 1-30, 1-20, 1-10, 1-5, 1-3, 1, 2, or 3) nucleotides at its 5'-end and/or 3'-end, and having the function of the nucleotide fragment with a sequence shown as SEQ ID NO: 16. The nucleotide sequence in b) may have a sequence identity of 80%, 81%, 82%, 83%, 84%, 85%, 86%, 87%, 88%, 89%, 90%, 91%, 92%, 93%, 94%, 95%, 96%, 97%, 98% or 99% or more to SEQ ID NO: 16.

The promoter of the present disclosure comprises a nucleic acid having a sequence identity of at least 80%, preferably having a sequence identity of at least 90%, preferably having a sequence identity of at least 95%, further preferably having a sequence identity of at least 98% to the nucleotide sequence shown as SEQ ID NO: 16; more further preferably, the promoter comprises the nucleotide sequence shown as SEQ ID NO: 16; particularly preferably, the promoter is the nucleotide sequence shown as SEQ ID NO: 16. The promoter may comprise, in addition to nucleotides having a sequence identity of at least 80% to the nucleotide sequence shown as SEQ ID NO: 16, base sequences at its terminals, such as restriction enzyme sites, att sequences for Gateway cloning and other sites for cloning into vectors.

In some embodiments, the specific promoter is Sox10, with its nucleotide sequence shown as SEQ ID NO: 16.

The present disclosure provides a primer pair D for amplifying the above specific promoter, comprising a forward primer and a reverse primer, the nucleotide sequence of the forward primer is shown as SEQ ID NO: 18; and the nucleotide sequence of the reverse primer is shown as SEQ ID NO: 19.

The present disclosure provides a cochlear supporting cell-specific recombinant vector, comprising the above specific promoter.

In some embodiments, the gene of the specific promoter Sox10 is inserted at a Kpn1/Mlu1 or Xbal/BamHI multiple cloning site of a basic vector. Preferably, the specific promoter Sox10 is inserted at the Xbal/BamHI multiple cloning site of the basic vector.

In some embodiments, the recombinant vector further comprises a target gene. In some embodiments, the target gene is located at the 3' end of the gene of the specific promoter. In some embodiments, the target gene is expressed in the cochlear supporting cells. In some embodiments, the target gene is selected from one or more of human deafness-causing genes, such as Prestin, otof, vglut3, and Gjb2.

The present disclosure provides a pharmaceutical composition, comprising the above recombinant vector.

The present disclosure provides a use of the specific promoter, the primer pair, the recombinant vector or the pharmaceutical composition, which is at least any one selected from:
1) use in preparing a medicine for preventing hearing-related diseases;
2) use in preparing a medicine for gene therapies of hearing-related diseases;
3) use in preparing and screening a medicine or vector for preventing and/or treating hearing-related diseases;
4) use in constructing animal models of hearing-related diseases;
5) use in constructing animal models of diseases accompanied with deafness genetic abnormalities;
6) use in in vitro studies related to cochlear supporting cell regeneration;
7) use in in vitro studies related to cochlear supporting cell damage protection;
8) use in in vitro studies related to inner ear development;
9) use in in vitro studies related to the pathogenesis of hearing-related diseases;
10) use in in vitro studies related to auditory synaptic reconstruction.

The present disclosure provides a method for treating hearing-related diseases, comprising: administering to a patient in need thereof an effective amount of the recombinant vector.

In one embodiment, the present disclosure provides a use of the cochlea-specific promoter Sox10 to specifically initiate the expression of an exogenous gene in cochlear supporting cells, in which the promoter Sox10 is loaded into an adeno-associated viral vector and infect the cochlear supporting cells of mice in vivo, with the results indicating that promoter Sox10 is capable of specifically initiating the expression of mNeonGreen gene (a kind of fluorescent protein) in cochlear supporting cells.

In one embodiment, the present disclosure provides a use of the specific promoter Sox10 in constructing transgenic mouse models, in which the promoter Sox10 is loaded into an adeno-associated viral vector, and at the same time, the target gene is constructed onto the adeno-associated viral vector, so that the transcription and expression of the target gene can be controlled so as to construct transgenic mouse models.

The promoter Sox10 of the present disclosure is the promoter which specifically acts in cochlear supporting cells, thereby enabling the gene operatively attached at the downstream of the promoter to be specifically expressed in cochlear supporting cells. The promoter of the present disclosure can be obtained by gene engineering. For example, the gene of the human promoter Sox10 is obtained from human genome by deletion, insertion or replacement of partial bases to get promoters having desired base sequences. Additionally, the promoters having desired base sequences can also be obtained by partial synthesis or total synthesis.

Compared with the prior art, the specific promoter Sox10 of the present disclosure has the following advantages and beneficial effects:
The present disclosure provides a hair cell-specific promoter Sox10, which can mediate the target gene to be specifically expressed in the cochlear and vestibular hair cells of mice, thereby labeling or manipulating the hair cells.

The specific promoter Sox10 of the present disclosure can reduce the expression of exogenous genes in other tissues, e.g., brain tissues, thereby improving the safety of gene therapies.

The specific promoter Sox10 of the present disclosure mediates the expression of the target gene, which is more flexible, more convenient in application and lower in cost than the traditional transgenic methods.

The specific promoter Sox10 of the present disclosure can be loaded into desired vectors, and at the same time, it can express therapeutic genes for gene therapies, which cannot be achieved with transgenic animals.

The specific promoter Sox10 of the present disclosure has extensive application value and market prospects in auditory neuroscience, modeling of deafness diseases, gene therapies and other fields.

### V. Cochlear type I spiral ganglion neuron-specific promoter Ngfr 1

The present disclosure provides a cochlear type I spiral ganglion neuron-specific promoter and use thereof. The promoter is a type I spiral ganglion neuron-specific promoter obtained from the genome of C57BL/6J strain mice, which can regulate the specific expression of genes in the type I spiral ganglion neurons. In the present disclosure, the promoter is loaded into an adeno-associated viral vector to infect the cochlea of mice in vivo, which can mediate the target gene to be specifically expressed in the cochlear type I spiral ganglion neurons, thereby labeling or manipulating the type I spiral ganglion neurons. Such a promoter has extensive application value and market prospects in terms of the structural and functional analysis of cochlear type I spiral ganglion neurons, modeling of diseases, gene therapies and the like.

One of the objectives of the present disclosure is to provide a cochlear type I spiral ganglion neuron-specific promoter,
a) the specific promoter comprises a nucleotide sequence shown as SEQ ID NO: 21; or
b) the specific promoter comprises a nucleotide fragment having a sequence identity of more than 80% to SEQ ID NO: 21 and having a function of the specific promoter defined in a).

In the present disclosure, the nucleotide fragment in b) specifically refers to: a nucleotide fragment obtained from substitution, deletion or addition of one or more (specifically, 1-50, 1-30, 1-20, 1-10, 1-5, 1-3, 1, 2, or 3) nucleotides to the nucleotide sequence shown as SEQ ID NO: 21, or from addition of one or more (specifically, 1-50, 1-30, 1-20, 1-10, 1-5, 1-3, 1, 2, or 3) nucleotides at its 5'-end and/or 3'-end, and having the function of the nucleotide fragment with a sequence shown as SEQ ID NO: 21. The nucleotide sequence in b) may have a sequence identity of 80%, 81%, 82%, 83%, 84%, 85%, 86%, 87%, 88%, 89%, 90%, 91%, 92%, 93%, 94%, 95%, 96%, 97%, 98% or 99% or more to SEQ ID NO: 21.

The promoter of the present disclosure comprises a nucleic acid having a sequence identity of at least 80%, preferably having a sequence identity of at least 90%, preferably having a sequence identity of at least 95%, further preferably having a sequence identity of at least 98% to the nucleotide sequence shown as SEQ ID NO: 21; more further preferably, the promoter comprises the nucleotide sequence shown as SEQ ID NO: 21; particularly preferably, the promoter is the nucleotide sequence shown as SEQ ID NO: 21. The promoter may comprise, in addition to nucleotides having a sequence identity of at least 80% to the nucleotide sequence shown as SEQ ID NO: 21, base sequences at its terminals, such as restriction enzyme sites, att sequences for Gateway cloning and other sites for cloning into vectors.

In some embodiments, the specific promoter is Ngfr 1, with its nucleotide sequence shown as SEQ ID NO: 21.

The present disclosure provides a primer pair E for amplifying the above specific promoter, comprising a forward primer and a reverse primer, the nucleotide sequence of the forward primer is shown as SEQ ID NO: 23; and the nucleotide sequence of the reverse primer is shown as SEQ ID NO: 24.

The present disclosure provides a cochlear type I spiral ganglion neuron-specific recombinant vector, comprising the above specific promoter.

In some embodiments, the gene of the specific promoter Ngfr 1 is inserted at a Kpn1/Mlu1 or Xbal/BamHI multiple cloning site of a basic vector. Preferably, the specific promoter Ngfr 1 is inserted at the Kpn1/Mlu1 multiple cloning site of the basic vector.

In some embodiments, the recombinant vector further comprises a target gene. In some embodiments, the target gene is located at the 3' end of the gene of the specific promoter. In some embodiments, the target gene is expressed in the cochlear type I spiral ganglion neurons. In some embodiments, the target gene is selected from one or more of human deafness-causing genes, such as Prestin, otof, vglut3, and Gjb2.

The present disclosure provides a pharmaceutical composition, comprising the above recombinant vector.

The present disclosure provides a use of the specific promoter, the primer pair, the recombinant vector or the pharmaceutical composition, which is at least any one selected from:
1) use in preparing a medicine for preventing hearing-related diseases;
2) use in preparing a medicine for gene therapies of hearing-related diseases;
3) use in preparing and screening a medicine or vector for preventing and/or treating hearing-related diseases;
4) use in constructing animal models of hearing-related diseases;
5) use in constructing animal models of diseases accompanied with deafness genetic abnormalities;
6) use in in vitro studies related to cochlear type I spiral ganglion neuron regeneration;
7) use in in vitro studies related to cochlear type I spiral ganglion neuron damage protection;
8) use in in vitro studies related to inner ear development;
9) use in in vitro studies related to the pathogenesis of hearing-related diseases;
10) use in in vitro studies related to auditory synaptic reconstruction.

The present disclosure provides a method for treating hearing-related diseases, comprising: administering to a patient in need thereof an effective amount of the recombinant vector.

In one embodiment, the present disclosure provides a use of the cochlea-specific promoter Ngfr 1 to specifically initiate the expression of an exogenous gene in cochlear type I spiral ganglion neurons, in which the promoter Ngfr 1 is loaded into an adeno-associated viral vector and infect the cochlear type I spiral ganglion neurons of mice in vivo, with the results indicating that promoter Ngfr 1 is capable of specifically initiating the expression of mNeonGreen gene (a kind of fluorescent protein) in cochlear type I spiral ganglion neurons.

In one embodiment, the present disclosure provides a use of the specific promoter Ngfr 1 in constructing transgenic mouse models, in which the promoter Ngfr 1 is loaded into an adeno-associated viral vector, and at the same time, the target gene is constructed onto the adeno-associated viral vector, so that the transcription and expression of the target gene can be controlled so as to construct transgenic mouse models.

The promoter Ngfr 1 of the present disclosure is the promoter which specifically acts in cochlear type I spiral ganglion neurons, thereby enabling the gene operatively attached at the downstream of the promoter to be specifically expressed in the cochlear type I spiral ganglion neurons. The promoter Ngfr 1 of the present disclosure can be obtained by gene engineering. For example, the gene of the human promoter Ngfr 1 is obtained from human genome by deletion, insertion or replacement of partial bases to get promoters having desired base sequences. Additionally, the promoters having desired base sequences can also be obtained by partial synthesis or total synthesis.

Compared with the prior art, the specific promoter Ngfr 1 of the present disclosure has the following advantages and beneficial effects:
The present disclosure provides a cochlear type I spiral ganglion neuron-specific promoter Ngfr 1, which can mediate the target gene to be specifically expressed in the cochlear and vestibular hair cells of mice, thereby labeling or manipulating the hair cells.

The specific promoter Ngfr 1 of the present disclosure can reduce the expression of exogenous genes in other tissues, e.g., brain tissues, thereby improving the safety of gene therapies.

The specific promoter Ngfr 1 of the present disclosure mediates the expression of the target gene, which is more flexible, more convenient in application and lower in cost than the traditional transgenic methods.

The specific promoter Ngfr 1 of the present disclosure can be loaded into desired vectors, and at the same time, it can express therapeutic genes for gene therapies, which cannot be achieved with transgenic animals.

The specific promoter Ngfr 1 of the present disclosure has extensive application value and market prospects in auditory neuroscience, modeling of deafness diseases, gene therapies and other fields.

### VI. Cochlear type II spiral ganglion neuron-specific promoter Ngfr 2

The present disclosure provides a cochlear type II spiral ganglion neuron-specific promoter and use thereof. The promoter is a type II spiral ganglion neuron-specific promoter obtained from the genome of C57BL/6J strain mice, which can regulate the specific expression of genes in the type II spiral ganglion neurons. In the present disclosure, the promoter is loaded into an adeno-associated viral vector to infect the cochlea of mice in vivo, which can mediate the target gene to be specifically expressed in the cochlear type II spiral ganglion neurons, thereby labeling or manipulating the type II spiral ganglion neurons. Such a promoter has extensive application value and market prospects in terms of the structural and functional analysis of cochlear type II spiral ganglion neurons, auditory neuroscience, modeling of deafness diseases, gene therapies and the like.

One of the objectives of the present disclosure is to provide a cochlea type II spiral ganglion neuron-specific promoter,
a) the specific promoter comprises a nucleotide sequence shown as SEQ ID NO: 26; or
b) the specific promoter comprises a nucleotide fragment having a sequence identity of more than 80% to SEQ ID NO: 26 and having a function of the specific promoter defined in a).

In the present disclosure, the nucleotide fragment in b) specifically refers to: a nucleotide fragment obtained from substitution, deletion or addition of one or more (specifically, 1-50, 1-30, 1-20, 1-10, 1-5, 1-3, 1, 2, or 3) nucleotides to the nucleotide sequence shown as SEQ ID NO: 26, or from addition of one or more (specifically, 1-50, 1-30, 1-20, 1-10, 1-5, 1-3, 1, 2, or 3) nucleotides at its 5'-end and/or 3'-end, and having the function of the nucleotide fragment with a sequence shown as SEQ ID NO: 26. The nucleotide sequence in b) may have a sequence identity of 80%, 81%, 82%, 83%, 84%, 85%, 86%, 87%, 88%, 89%, 90%, 91%, 92%, 93%, 94%, 95%, 96%, 97%, 98% or 99% or more to SEQ ID NO: 26.

The promoter of the present disclosure comprises a nucleic acid having a sequence identity of at least 80%, preferably having a sequence identity of at least 90%, preferably having a sequence identity of at least 95%, further preferably having a sequence identity of at least 98% to the nucleotide sequence shown as SEQ ID NO: 26; more further preferably, the promoter comprises the nucleotide sequence shown as SEQ ID NO: 26; particularly preferably, the promoter is the nucleotide sequence shown as SEQ ID NO: 26. The promoter may comprise, in addition to nucleotides having a sequence identity of at least 80% to the nucleotide sequence shown as SEQ ID NO: 26, base sequences at its terminals, such as restriction enzyme sites, att sequences for Gateway cloning and other sites for cloning into vectors.

In some embodiments, the specific promoter is Ngfr 2, with its nucleotide sequence shown as SEQ ID NO: 26.

The present disclosure provides a primer pair for amplifying the above specific promoter F, comprising a forward primer and a reverse primer, the nucleotide sequence of the forward primer is shown as SEQ ID NO: 28; the nucleotide sequence of the reverse primer is shown as SEQ ID NO: 29.

The present disclosure provides a cochlear type II spiral ganglion neuron-specific recombinant vector, comprising the above specific promoter.

In some embodiments, the gene of the specific promoter Ngfr 2 is inserted at a Kpn1/Mlu1 or Xbal/BamHI multiple cloning site of a basic vector. Preferably, the specific promoter Ngfr 2 is inserted at the Xbal/BamHI multiple cloning site of the basic vector.

In some embodiments, the recombinant vector further comprises a target gene. In some embodiments, the target gene is located at the 3' end of the gene of the specific promoter. In some embodiments, the target gene is expressed in the cochlear type II spiral ganglion neurons. In some embodiments, the target gene is selected from one or more of human deafness-causing genes, such as Prestin, otof, vglut3, and Gjb2.

The present disclosure provides a pharmaceutical composition, comprising the above recombinant vector.

The present disclosure provides a use of the specific promoter, the primer pair, the recombinant vector or the pharmaceutical composition, which is at least any one selected from:
1) use in preparing a medicine for preventing hearing-related diseases;
2) use in preparing a medicine for gene therapies of hearing-related diseases;
3) use in preparing and screening a medicine or vector for preventing and/or treating hearing-related diseases;
4) use in constructing animal models of hearing-related diseases;
5) use in constructing animal models of diseases accompanied with deafness genetic abnormalities;
6) use in in vitro studies related to cochlear type II spiral ganglion neuron regeneration;
7) use in in vitro studies related to cochlear type II spiral ganglion neuron damage protection;
8) use in in vitro studies related to inner ear development;
9) use in in vitro studies related to the pathogenesis of hearing-related diseases;
10) use in in vitro studies related to auditory synaptic reconstruction.

The present disclosure provides a method for treating hearing-related diseases, comprising: administering to a patient in need thereof an effective amount of the recombinant vector.

In one embodiment, the present disclosure provides a use of the cochlea-specific promoter Ngfr 2 to specifically initiate the expression of an exogenous gene in cochlear type II spiral ganglion neurons, in which the promoter Ngfr 2 is loaded into an adeno-associated viral vector and infect the cochlear type II spiral ganglion neurons of mice in vivo, with the results indicating that promoter Ngfr 2 is capable of specifically initiating the expression of mNeonGreen gene (a kind of fluorescent protein) in cochlear type II spiral ganglion neurons.

In one embodiment, the present disclosure provides a use of the specific promoter Ngfr 2 in constructing transgenic mouse models, in which the promoter Ngfr 2 is loaded into an adeno-associated viral vector, and at the same time, the target gene is constructed onto the adeno-associated viral vector, so that the transcription and expression of the target gene can be controlled so as to construct transgenic mouse models.

The promoter Ngfr 2 of the present disclosure is the promoter which specifically acts in cochlear type II spiral ganglion neurons, thereby enabling the gene operatively attached at the downstream of the promoter Ngfr 2 to be specifically expressed in the cochlear type II spiral ganglion neurons. The promoter Ngfr 2 of the present disclosure can be obtained by gene engineering. For example, the gene of the human promoter Ngfr 2 is obtained from human genome by deletion, insertion or replacement of partial bases to get promoters having desired base sequences. Additionally, the promoters having desired base sequences can also be obtained by partial synthesis or total synthesis.

Compared with the prior art, the specific promoter Ngfr 2 of the present disclosure has the following advantages and beneficial effects:
The present disclosure provides a cochlear type II spiral ganglion neuron-specific promoter Ngfr 2, which can mediate the target gene to be specifically expressed in the cochlear and vestibular hair cells of mice, thereby labeling or manipulating the hair cells.

The specific promoter Ngfr 2 of the present disclosure can reduce the expression of exogenous genes in other tissues, e.g., brain tissues, thereby improving the safety of gene therapies.

The specific promoter Ngfr 2 of the present disclosure mediates the expression of the target gene, which is more flexible, more convenient in application and lower in cost than the traditional transgenic methods.

The specific promoter Ngfr 2 of the present disclosure can be loaded into desired vectors, and at the same time, it can express therapeutic genes for gene therapies, which cannot be achieved with transgenic animals.

The specific promoter Ngfr 2 of the present disclosure has extensive application value and market prospects in auditory neuroscience, modeling of deafness diseases, gene therapies and other fields.

### VII. Vestibular hair cell-specific promoter

The present disclosure provides a vestibular hair cell-specific promoter and use thereof. The promoter is a vestibular hair cell-specific promoter obtained from the genome of C57BL/6J strain mice, which can regulate the specific expression of genes in the vestibular hair cells. In the present disclosure, the promoter is loaded into an adeno-associated viral vector to infect the cochlea of mice in vivo, which can mediate the target gene to be specifically expressed in the vestibular hair cells, thereby labeling or manipulating the vestibular hair cells. Such a promoter has extensive application value and market prospects in terms of the structural and functional analysis of vestibular hair cells, auditory neuroscience, modeling of deafness diseases, gene therapies and the like.

One of the objectives of the present disclosure is to provide a vestibular hair cell-specific promoter,
a) the specific promoter comprises a nucleotide sequence shown as SEQ ID NO: 31; or
b) the specific promoter comprises a nucleotide fragment having a sequence identity of more than 80% to SEQ ID NO: 31 and having a function of the specific promoter defined in a).

In the present disclosure, the nucleotide fragment in b) specifically refers to: a nucleotide fragment obtained from substitution, deletion or addition of one or more (specifically, 1-50, 1-30, 1-20, 1-10, 1-5, 1-3, 1, 2, or 3) nucleotides to the nucleotide sequence shown as SEQ ID NO: 31, or from addition of one or more (specifically, 1-50, 1-30, 1-20, 1-10, 1-5, 1-3, 1, 2, or 3) nucleotides at its 5'-end and/or 3'-end, and having the function of the nucleotide fragment with a sequence shown as SEQ ID NO: 31. The nucleotide sequence in b) may have a sequence identity of 80%, 81%, 82%, 83%, 84%, 85%, 86%, 87%, 88%, 89%, 90%, 91%, 92%, 93%, 94%, 95%, 96%, 97%, 98% or 99% or more to SEQ ID NO: 31.

The promoter of the present disclosure comprises a nucleic acid having a sequence identity of at least 80%, preferably having a sequence identity of at least 90%, preferably having a sequence identity of at least 95%, further preferably having a sequence identity of at least 98% to the nucleotide sequence shown as SEQ ID NO: 31; more further preferably, the promoter comprises the nucleotide sequence shown as SEQ ID NO: 31; particularly preferably, the promoter is the nucleotide sequence shown as SEQ ID NO: 31. The promoter may comprise, in addition to nucleotides having a sequence identity of at least 80% to the nucleotide sequence shown as SEQ ID NO: 31, base sequences at its terminals, such as restriction enzyme sites, att sequences for Gateway cloning and other sites for cloning into vectors.

In some embodiments, the specific promoter is Dnah5, with its nucleotide sequence shown as SEQ ID NO: 31.

The present disclosure provides a primer pair G for amplifying the above specific promoter, comprising a forward primer and a reverse primer, the nucleotide sequence of the forward primer is shown as SEQ ID NO: 33; and the nucleotide sequence of the reverse primer is shown as SEQ ID NO: 34.

The present disclosure provides a vestibular hair cell-specific recombinant vector, comprising the above specific promoter.

In some embodiments, the gene of the specific promoter Dnah5 is inserted at a Kpn1/Mlu1 or Xbal/BamHI multiple cloning site of a basic vector. Preferably, the specific promoter Dnah5 is inserted at the Kpn1/Mlu1 multiple cloning site of the basic vector.

In some embodiments, the recombinant vector further comprises a target gene. In some embodiments, the target gene is located at the 3' end of the gene of the specific promoter. In some embodiments, the target gene is expressed in the cochlear and/or vestibular cell. In some embodiments, the target gene is selected from one or more of human deafness-causing genes, such as Prestin, otof, vglut3, and Gjb2.

The present disclosure provides a pharmaceutical composition, comprising the above recombinant vector.

The present disclosure provides a use of the specific promoter, the primer pair, the recombinant vector or the pharmaceutical composition, which is at least any one selected from:
1) use in preparing a medicine for preventing hearing-related diseases;
2) use in preparing a medicine for gene therapies of hearing-related diseases;
3) use in preparing and screening a medicine or vector for preventing and/or treating hearing-related diseases;
4) use in constructing animal models of hearing-related diseases;
5) use in constructing animal models of diseases accompanied with deafness genetic abnormalities;
6) use in in vitro studies related to vestibular hair cell regeneration;
7) use in in vitro studies related to vestibular hair cell damage protection;
8) use in in vitro studies related to inner ear development;
9) use in in vitro studies related to the pathogenesis of hearing-related diseases;
10)use in in vitro studies related to auditory synaptic reconstruction.

The present disclosure provides a method for treating hearing-related diseases, comprising: administering to a patient in need thereof an effective amount of the recombinant vector.

In one embodiment, the present disclosure provides a use of the specific promoter Dnah5 to specifically initiate the expression of an exogenous gene in vestibular hair cells, in which the promoter Dnah5 is loaded into an adeno-associated viral vector and infect the cochlear and vestibular hair cells of mice in vivo, with the results indicating that promoter Dnah5 is capable of specifically initiating the expression of mNeonGreen gene (a kind of fluorescent protein) in vestibular hair cells.

In one embodiment, the present disclosure provides a use of the specific promoter Dnah5 in constructing transgenic mouse models, in which the promoter Dnah5 is loaded into an adeno-associated viral vector, and at the same time, the target gene is constructed onto the adeno-associated viral vector, so that the transcription and expression of the target gene can be controlled so as to construct transgenic mouse models.

The promoter Dnah5 of the present disclosure is the promoter which specifically acts in vestibular hair cells, thereby enabling the gene operatively attached at the downstream of the promoter Dnah5 to be specifically expressed in the vestibular hair cells. The promoter Dnah5 of the present disclosure can be obtained by gene engineering. For example, the gene of the human promoter Dnah5 is obtained from human genome by deletion, insertion or replacement of partial bases to get promoters having desired base sequences. Additionally, the promoters having desired base sequences can also be obtained by partial synthesis or total synthesis.

Compared with the prior art, the specific promoter Dnah5 of the present disclosure has the following advantages and beneficial effects:
The present disclosure provides a vestibular hair cell-specific promoter Dnah5, which can mediate the target gene to be specifically expressed in the cochlear and vestibular hair cells of mice, thereby labeling or manipulating the hair cells.

The specific promoter Dnah5 of the present disclosure can reduce the expression of exogenous genes in other tissues, e.g., brain tissues, thereby improving the safety of gene therapies.

The specific promoter Dnah5 of the present disclosure mediates the expression of the target gene, which is more flexible, more convenient in application and lower in cost than the traditional transgenic methods.

The specific promoter Dnah5 of the present disclosure can be loaded into desired vectors, and at the same time, it can express therapeutic genes for gene therapies, which cannot be achieved with transgenic animals.

The specific promoter Dnah5 of the present disclosure has extensive application value and market prospects in auditory neuroscience, modeling of deafness diseases, gene therapies and other fields.

### BRIEF DESCRIPTION OF THE DRAWINGS

FIG. 1 shows a diagram of pAAV-CMVen-STRC promoter-NLS-mNeonGreen-WPRE- SV40 expression vector.

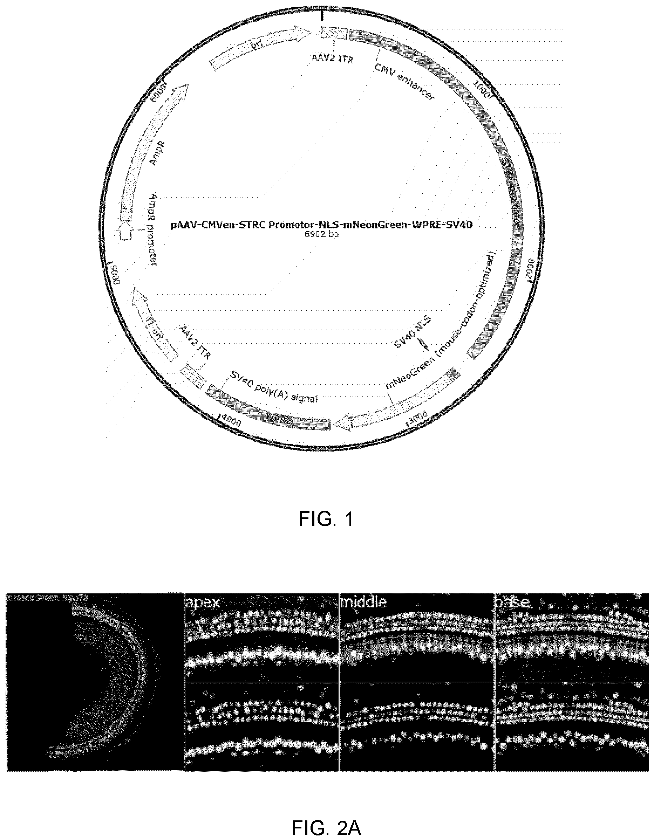

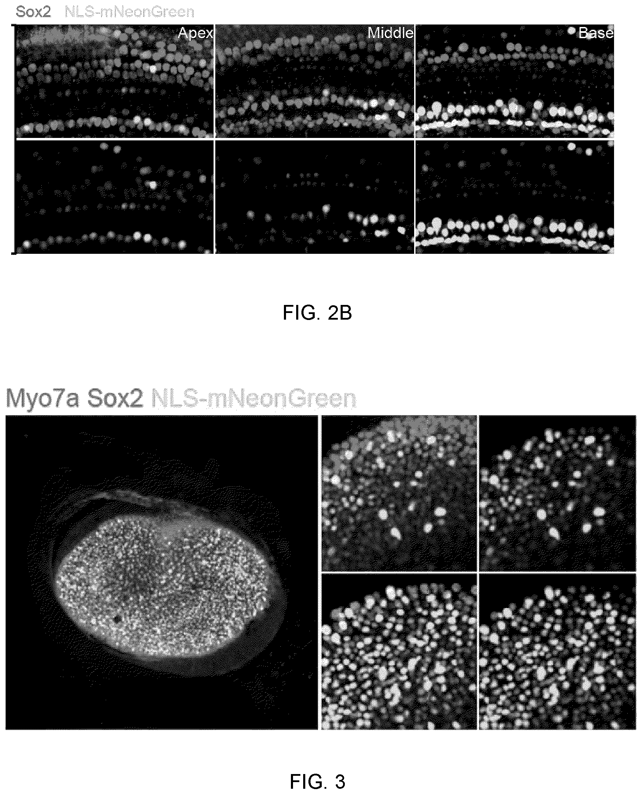

FIG. 2 shows a smear slide diagram of cochlea injected with rAAV-CMVen-STRC promoter-NLS-mNeonGreen-WPRE- SV40 virus. The FIG. 2 shows that an STRC promoter can specifically initiate the expression of genes in the cochlear hair cells. In FIG. 2A, purple indicates a marker protein Myo7a of hair cells, green indicates virus-infected cells, which specifically express fluorescent proteins in cochlear hair cells. In FIG. 2B, red indicates a marker Sox2 of supporting cells, green indicates virus-infected cells, with the results indicating that the STRC promoter can reduce the expression in supporting cells.
FIG. 3 shows a smear slide diagram of vestibular utricle injected with rAAV-CMVen-STRC promoter-NLS-mNeonGreen-WPRE- SV40 virus. The FIG. 3 shows that the STRC promoter can specifically initiate the expression of genes in the vestibular hair cells. In FIG.3, purple indicates a marker protein Myo7a of hair cells, red indicates a marker protein Sox2 of supporting cells, green indicates virus-infected cells, with the results indicating that the STRC promoter can specifically express fluorescent protein in vestibular hair cells, but not expresses in supporting cells other than hair cells.
FIG. 4 shows a smear slide diagram of cochlea injected with rAAV-CMVen-STRC mini promoter-NLS-mNeonGreen-WPRE- SV40 virus, in which purple indicates a marker protein Myo7a of hair cells, red indicates a marker Sox2 of supporting cells, green indicates virus-infected cells, with the results indicating that the STRC mini promoter can regulate the fluorescent protein to be specifically expressed in the hair cells, while reducing the expression thereof in supporting cells.

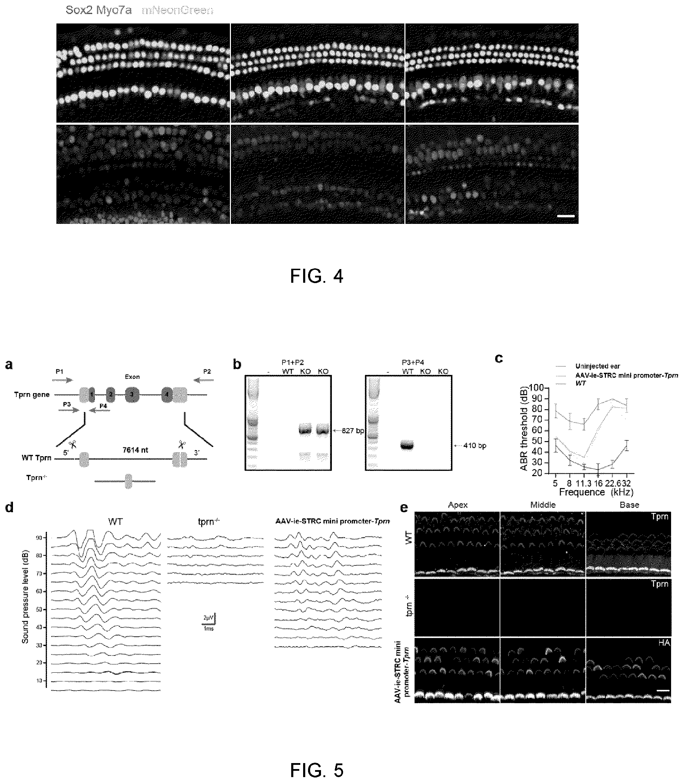

FIG. 5 shows a diagram showing the hearing restoration of mice with rAAV-CMVen-STRC mini promoter-Tprn. (a) Schematic diagram of Tprn KO mice. Using Crispr Cas9 technology, the exon 1-4 region, approximately 7614 nt bases, was excised. The genotype of the Tprn KO mice can be identified using two pairs of primers: P1, P2 and P3, P4. (b) PCR gel electrophoresis image for identification of the Tprn KO mouse tail. WT: normal mice; KO: Tprn KO mice; -: ddH2O. (c) Hearing test threshold graph. Black: normal mice; red: Tprn KO mice; green: mice treated by injection of rAAV-CMVen-STRC mini promoter-Tprn-WPRE- SV40. (d) Hearing test waveform graph at 11.3 kHz. Black: normal mice, with a threshold of 15 dB; red: Tprn KO mice, with a threshold of 75 dB; green: mice treated by injection of rAAV-CMVen-STRC mini promoter-Tprn-WPRE- SV40, with a threshold of 35 dB. (e) confocal imaging of normal mice, Tprn KO mice, mice treated by injection of rAAV-CMVen-STRC mini promoter-Tprn-WPRE- SV40. The results showed that rAAV-CMVen-STRC mini promoter-Tprn-WPRE- SV40 can be used for the expression of TPRN protein in hair cells and the restoration of hearing in mice.
FIG. 6 shows the smear slide diagrams of cochlea (a), vestibule (b) and spiral ganglion neurons (c) of mice injected with rAAV-CAG-NLS-mNeonGreen-WPRE- SV40 virus, and d shows the infection efficiencies of different kinds of cochlear cells. In FIGs. a and b, purple indicates a marker protein Myo7a of hair cells, red indicates a marker protein Sox2 of supporting cells, and green indicates virus-infected cells; in FIG. c, purple indicates a marker protein Tuj1 of spiral ganglion neurons, green indicates virus-infected cells.
FIG. 7 shows the sequence comparison results of the conservative degree of Slc26a5 in Embodiment 2.
FIG. 8 shows a diagram of the pAAV-Slc26a5 promoter-SHH mini promoter-NLS-mNeonGreen-WPRE-SV40 plasmid.
FIG. 9 shows a smear slide diagram of cochlea injected with rAAV-Slc26a5 promoter-SHH mini promoter-NLS-mNeonGreen-WPRE-SV40 virus, indicating that the Slc26a5 promoter can specifically initiate the expression of genes in the cochlear outer hair cells.
FIG. 10 shows a diagram of the pAAV- otof promoter-SHH mini promoter-NLS-mNeonGreen-WPRE-SV40 plasmid in Embodiment 3.
FIG. 11 shows a smear slide diagram of cochlea injected with rAAV-otof promoter-SHH mini promoter-NLS-mNeonGreen-WPRE-SV40 virus in Embodiment 3, indicating that the otof promoter can specifically initiate the expression of genes in cochlear inner hair cells.
FIG. 12 shows a diagram of pAAV-Sox10 promoter-SHH mini promoter-NLS-mNeonGreen-WPRE-SV40 plasmid.
FIG. 13 shows a smear slide diagram of cochlea injected with rAAV- Sox10 promoter-SHH mini promoter-NLS-mNeonGreen-WPRE-SV40 virus, indicating that the Sox10 promoter can specifically initiate the expression of genes in the cochlear supporting cells. FIG. 13A shows chromogenic results of the supporting cells; and FIG. 13B shows the chromogenic results of the hair cells.
FIG. 14 shows the conserved domains of the Ngfr gene sequence in different species, in which the Ngfr 1 promoter sequence is circled in red.
FIG. 15 shows a diagram of pAAV- Ngfr 1 promoter-SHH mini promoter-NLS-mNeonGreen-WPRE-SV40 plasmid.
FIG. 16 shows a slice diagram of cochlea injected with rAAV- Ngfr 1 promoter-SHH mini promoter-NLS-mNeonGreen-WPRE-SV40 virus, indicating that the Ngfr 1 promoter can specifically initiate the expression of genes in SGNs, but not in type II spiral ganglion neurons.
FIG. 17 shows a slice diagram of cochlea injected with rAAV- Ngfr 1 promoter-SHH mini promoter-NLS-mNeonGreen-WPRE-SV40 virus, indicating that the Ngfr 1 promoter can specifically initiate the expression of genes in type I spiral ganglion neurons.
FIG. 18 shows the conserved domains of the Ngfr gene sequence in different species, in which the Ngfr 2 promoter sequence is circled in red.
FIG. 19 shows a diagram of pAAV- Ngfr 2 promoter-SHH mini promoter-NLS-mNeonGreen-WPRE-SV40 plasmid.
FIG. 20 shows a slice diagram of cochlea injected with rAAV- Ngfr 2 promoter-SHH mini promoter-NLS-mNeonGreen-WPRE-SV40 virus, indicating that the Ngfr 2 promoter can specifically initiate the expression of genes in SGNs and type II spiral ganglion neurons.
FIG. 21 shows a slice diagram of cochlea injected with rAAV- Ngfr 2 promoter-SHH mini promoter-NLS-mNeonGreen-WPRE-SV40 virus, indicating that the Ngfr 2 promoter cannot specifically initiate the expression of genes in type I spiral ganglion neurons.
FIG. 22 shows a diagram of pAAV-CMVen-Dnah5 promoter-NLS-mNeonGreen-WPRE-SV40 plasmid.
FIG. 23 shows smear slide diagrams of vestibule (a) and cochlea (b) injected with rAAV-CMVen-Dnah5 promoter-NLS-mNeonGreen-WPRE-SV40 virus, indicating that the Dnah5 promoter can specifically initiate the expression of genes in the cochlear outer hair cells.

### DETAILED DESCRIPTION

The present disclosure provides a cochlear and/or vestibular cell-specific promoter and use thereof. The promoter can regulate the specific expression of genes in particular cochlear and/or vestibular cells. In the present disclosure, the promoter is loaded into an adeno-associated viral vector to infect the cochlea and/or vestibule of mice in vivo, which can mediate the target gene to be specifically expressed in the cochlear and/or vestibular hair cells, thereby labeling or manipulating the cochlear and/or vestibular cells. Such a promoter has extensive application value and market prospects in terms of the structural and functional analysis of cochlear and/or vestibular cells, auditory neuroscience, modeling of deafness diseases, gene therapies and the like.

In the present disclosure, there is no limitation on the type of vectors, which may be viral vectors, including, but not limited to, one or more of adeno-associated viral vectors, adenoviral vectors, lentiviral vectors, retroviral vectors, herpes simplex viral vectors, herpes simplex viral amplicon vectors, pseudorabies viral vectors, baculoviral vectors, poxviral vectors. In some embodiments, the vector is an adeno-associated viral vector.

In the present disclosure, the diseases are hearing-related diseases; preferably, the sensorineural hearing loss is selected from presbycusis, noise-induced hearing loss, ototoxic drug-associated hearing loss, sudden hearing loss, ischemic deafness,, autoimmune deafness, Meniere's disease, head injury-associated hearing loss, hereditary hearing loss, dysfunction of organ of Corti due to other or unknown causes, or damage to organ of Corti caused by viral or bacterial infection, or a combination thereof.

In the present disclosure, the pharmaceutical composition may further comprises a pharmaceutically acceptable carrier. The carrier may comprise a variety of excipients and diluents which are not themselves necessary active ingredients and are not excessively toxic after administration. Suitable carriers should be well known to those skilled in the art. The acceptable carriers are, e.g., sterile water or normal saline, stabilizers, excipients, antioxidants (ascorbic acid, etc.), buffering agents (phosphoric acid, citric acid, and other organic acids), preservatives, surfactants (PEG, Tween, etc.), chelating agents (EDTA, etc.), binding agents, and the like. In addition, they can also contain other low molecular weight polypeptides; proteins such as serum albumin, gelatin or immunoglobulin; amino acids such as glycine, glutamine, asparagine, arginine and lysine; saccharides or carbohydrates such as polysaccharide and monosaccharide; sugar alcohols such as mannitol or sorbitol. When being used in the preparation of aqueous solution for injection, for example, normal saline, isotonic solution containing glucose or other auxiliary drugs, such as D-sorbitol, D-mannose, D-mannitol, sodium chloride, can be combined with appropriate solubilizers, such as alcohols (ethyl alcohol, etc.), polyols (propylene glycol, polyethylene glycol (PEG), etc.), nonionic surfactants (Tween 80, HCO-50), and the like.

### Embodiment 1 Promoter STRC

### 1. Isolation and identification of full-length promoter STRC

### a. Design of the primer

Based on the whole genome sequence of the C57BL/6J mouse strain provided by NCBI, an amplification primer was designed according to the sequence of the STRC gene in mice, and according to the selected vector and the characteristics of the target gene, a homologous arm was added before the primer for the recombinant synthesis of a plasmid. The primer sequences are as below: upstream primer: ctattaccatggtgctcgagacaatatcagttcttgaggccaagtag (SEQ ID NO: 3); downstream primer: gcccagtgtgcacctggaaatggtacctcgacggtaagtcc (SEQ ID NO: 4). Both the upstream primer and the downstream primer carry homologous arm sequences, which are underlined respectively. The primer was synthesized by Shanghai GENEWIZ Biotechnology Co., Ltd.

### b. Cloning of the promoter STRC

With the genomic DNA of the C57BL/6J mouse strain as the template, STRC promoter fragments were amplified by PCR using upstream and downstream primers. The amplification conditions for PCR were: predegeneration at 95°C for 5 min; 30 cycles of degeneration at 95°C for 30 s, annealing at 59°C for 30 s, and extension at 72°C for 1.5 min; extension at 72°C for 10 min. After confirmation of the PCR amplification product by gel electrophoresis with 1% agarose, the target fragments were recovered by gel slicing and sent to BioSune Technology (Shanghai, China) for sequencing. The results showed that: the amplified sequence was the STRC promoter sequence, with its nucleotide sequence shown as SEQ ID NO: 1. The recovered fragments were detected by Nanodrop2000 for the concentration.

SEQ ID NO: 1

acaatatcagttcttgaggccaagtagctgctgttggcaaggattaactactgtcatgttttgac

cagggtctgcaccaccctagggaacttcaagttccctatgaggctattgtcaagaccaatagccacttg

actcaacttgtggaaatagtgttcacaaagtttccatattaggaacttaccaatcatcttgtaaaaaccta

caccaaagtgtctgggcagagaacctaggctctggctatggttacaatagagggcatatgtatattttga

tttttttttttttgagatggtttcactatgtaaccctggctagcccgaacttgttatatagacaaggctggactc

agactcacagagatcatcctgcctctgcctcttaagtgctgggattaaaagcctgtgccaccattcccag
cccaacacatttgtttgcttgagaaagggtttctctgtgtagcccaggatgtcctgaaactcactctgt
agactaggctggtctcaaactcagtgagccacctgcctttacctcctgagtgctgctgttaaaggcatgt
gccaccaccatctagcttgattttttaattaatttttaaaaacgtgtagtatacatgtaatggtgtgttccttt
aatcccagcactcaggctgacagaggcaggcagagctctgtgagtttaaggccagcctaatctacat
agagaattccaccgcagccagggctacctggggagaccctatctcaatgatacgatgatgatggatg
acttgactgaatggatatctgtgcaccatgtgcatacctggttccctcagagaccagaagaggaggtca
gatgccctgaactggaattagagatggttgtgagcagccatatgaatgctgggaatcaaacccaggtc
ctctgggtttggaagagcagcagtcactgttctaatctgctgagccatccctctagacctgctcataaggt
ttttttatgcaagaagagatgaccagtctctggacagaccagcttatattgaatgaaatctttctaacccat
gaataggattccatgtgtagagagttttaacttgagaaagaaaaagaaactgcaatgtttacatggcct
aatcaagtaacagctctctgagtctctctccttacctgttaattgaagggtttacctatgtggtttgtgaagtc
ctgtctactccttaatattgcaggattccaaggatttatttttatgtgtgtatgtgtgttgtatctgtgtaggcatg
ggcaggttaagtgcagacgcccacagaggcaagagacatcagattcccctggagctggagttacag
gcagttctgaaccacccaattcaggtgcatgtaactcaactcaggtcatctgcaaaggcagcatacac
tctcagctgctgagccatctcttcagcaccaatactgtgggactctggatagtgtgtctaaggtaacaact
ctcctcttcctaaatagtctacccttgaaacatctatcttaatatcttctcgctgcccactgctggctgccag
accatttctcaaattcttcaaatctcagttgtctctcaaactccttttgtactttctttgtttgatcacatttttctgta
gacccccagattatatttttagtctgccttcttctctctggctcttaactcatgccgtctccttgggtaatactct
gagggccccgagatcccctgtcattggtccctctaacagaagtacacccttctcccaaagccagagg
atcaggtgttcagtggctccggtgacaggtgtgatgtcaggttacagatactgtttcctgagagaccaca
cgacagtgtcagaggaaccccagcctgcccacaatgccccgtcttcacctggctatcccttcatggtga
gcatagccagaactcacctctaggcccagtgtgcacctggaaatggtacctcgac

### 2. Functional identification of full-length promoters STRC

### a. Construction of a recombinant expression vector

First, a CMV enhancer sequence was synthesized, and a homologous arm linked to the vector was added before and after the sequence. The homologous arm was underlined, and its nucleotide sequence was shown as SEQ ID NO: 5.

SEQ ID NO: 5

gttcctgcggccgcacgcgtgacattgattattgactagttattaatagtaatcaattacggggt

cattagttcatagcccatatatggagttccgcgttacataacttacggtaaatggcccgcctggctgacc

gcccaacgacccccgcccattgacgtcaataatgacgtatgttcccatagtaacgccaatagggacttt

ccattgacgtcaatgggtggactatttacggtaaactgcccacttggcagtacatcaagtgtatcatatgc

caagtacgccccctattgacgtcaatgacggtaaatggcccgcctggcattatgcccagtacatgacct

tatgggactttcctacttggcagtacatctacgtattagtcatcgctattaccatggtgctcgag

By means of multi-fragment homologous recombination, the promoter CMV enhancer fragment and the STRC fragment were linked into pAAV-CAG-mNeonGreen-WPRE-SV40 plasmid digested with two enzymes, Kpn1 and Mlu1, using a recombination kit (Vazyme, MultiS One Step Cloning Kit) by means of homologous recombination. The recombination system consists of: a linearized pAAV-CAG-mNeonGreen-WPRE-SV40 vector, 50 ng; clonal STRC promoter fragment, 30 ng; synthetic CMV enhancer fragment, 30 ng; a recombinant ligase Exnase MultiS, 1 µL; a recombinant linking buffer 5x CE MultiS Buffer, 4 µL; ddH2O, supplement to 20 µL; reaction at 50°C for 20 min, to give the recombinant product.

The ligation product was transformed into Escherichia Coli JM109 competent cells and placed in an incubator at 37°C overnight. Single colonies were picked for colony PCR identification. Positive colonies were subjected to expanded culture and plasmid extraction, followed by sequencing to verify the plasmid. The correctly sequenced plasmid was named as pAAV-CMVen-STRC promoter-NLS-mNeonGreen-WPRE-SV40. The diagram of the constructed plasmid expression vector is shown in FIG. 1.

### b. Virus production from a recombinant expression vector

The recombinant expression vector was virally packaged using the traditional triple plasmid packaging adeno-associated virus method. The rep-cap plasmid with AAV-ie capsid, pAAV-CMVen-STRC promoter-NLS-mNeonGreen-WPRE- SV40 and pHelper plasmid 3 (same as SEQ ID NO: 12 in a patent document to AAV-ie (CN 110437317A)) were co-transfected in HEK-293T cells in appropriate amounts, concentrated and purified by iodixanol gradient centrifugation, and finally detected by SYBR Green qPCR method for the titer of the recombinant adeno-associated virus. The titer of the finally obtained rAAV-CMVen-STRC promoter-NLS-mNeonGreen-WPRE- SV40 virus was 1×1013 vg/mL.

### c. In vivo verification of the full-length STRC promoter

The rAAV-CMVen-STRC promoter-NLS-mNeonGreen-WPRE-SV40 virus was injected into the cochlea of a 2-3-day-old C57BL/6J mouse through the round window. After the virus was fully expressed for 10-14 days, the mouse was executed by cervical dislocation. The injected cochlea was removed and placed in 4% paraformaldehyde and fixed at room temperature for 2 hours, washed with PBS three times, for 5 min each time, then decalcified with 0.5 mM EDTA, and soaked at room temperature for 2 hours to soften the cochlea. The basilar membrane was dissected under a stereo microscope with an ophthalmic scalpel into three parts: the apex, middle, and base, and the vestibular utricle tissue. By means of immunofluorescence staining, hair cells were labeled with anti-Myo7a antibodies and supporting cells were labeled with anti-Sox2 antibodies to prepare cochlea samples. The cochlea samples were imaged with a laser confocal scanning microscope, showing that virus-infected cells would express green fluorescent protein in their nuclei. The wavelength of the excitation light was 488 nm, 561 nm, 647 nm, respectively. The in vivo assay results are shown in FIGs. 2 and 3, where the green fluorescence is substantially completely co-labeled with the hair cell marker Myo7a shown in purple, indicating that the STRC promoter can specifically initiate the expression of genes in the utricular hair cells of the cochlea and vestibule.

### 3. Functional identification of the shortened STRC promoter sequence

The shortened STRC promoter sequence is also specific, referred to as STRC mini promoter, with its sequence shown below (SEQ ID NO: 2):

gtgctcgagagcagccatatgaatgctgggaatcaaacccaggtcctctgggtttggaaga
gcagcagtcactgttctaatctgctgagccatccctctagacctgctcataaggtttttttatgcaagaaga
gatgaccagtctctggacagaccagcttatattgaatgaaatctttctaacccatgaataggattccatgt
gtagagagttttaacttgagaaagaaaaagaaactgcaatgtttacatggcctaatcaagtaacagct
ctctgagtctctctccttacctgttaattgaagggtttacctatgtggtttgtgaagtcctgtctactccttaatat
tgcaggattccaaggatttatttttatgtgtgtatgtgtgttgtatctgtgtaggcatgggcaggttaagtgca
gacgcccacagaggcaagagacatcagattcccctggagctggagttacaggcagttctgaaccac
ccaattcaggtgcatgtaactcaactcaggtcatctgcaaaggcagcatacactctcagctgctgagcc
atctcttcagcaccaatactgtgggactctggatagtgtgtctaaggtaacaactctcctcttcctaaatag
tctacccttgaaacatctatcttaatatcttctcgctgcccactgctggctgccagaccatttctcaaattctt
caaatctcagttgtctctcaaactccttttgtactttctttgtttgatcacatttttctgtagacccccagattatat
ttttagtctgccttcttctctctggctcttaactcatgccgtctccttgggtaatactctgagggccccgagatc
ccctgtcattggtccctctaacagaagtacacccttctcccaaagccagaggatcaggtgttcagtggct
ccggtgacaggtgtgatgtcaggttacagatactgtttcctgagagaccacacgacagtgtcagagga
accccagcctgcccacaatgccccgtcttcacctggctatcccttcatggtgagcatagccagaactca
cctctaggcccagtgtgcacctggaaat

### a. Construction of STRC mini promoter-containing virus

According to the construction method in a and b of 2 in Embodiment 1, rAAV-CMVen-STRC mini promoter-NLS-mNeonGreen-WPRE- SV40 virus was obtained only by replacing the STRC promoter with a STRC mini promoter.

b. The in vivo verification results of mice are shown below:
The operation method was the same as that in c of 2 in Embodiment 1.

FIG. 4 shows a smear slide diagram of cochlea injected with rAAV-CMVen-STRC mini promoter-NLS-mNeonGreen-WPRE-SV40 virus, in which purple indicates a marker protein Myo7a of hair cells, red indicates a marker Sox2 of supporting cells, green indicates virus-infected cells, with the results indicating that the STRC mini promoter can regulate the fluorescent protein to be specifically expressed in the hair cells, while reducing the expression thereof in supporting cells.

### 5. Detection of the effect on hearing restoration using STRC mini promoter-packaged murine tprn genes

pAAV-CMVen-STRC mini promoter-tprn-WPRE- SV40 plasmid was constructed using STRC mini promoter-packaged murine tprn genes. The plasmid was packaged as rAAV-CMVen-STRC mini promoter-tprn-WPRE-SV40 virus by the above method and injected with P3 tprn-/- mice. 30 days after birth, the hearing of mice was tested by auditory brainstem response. The results showed that, hearing damage was severe in tprn-/- mice 30 days after birth, whereas mice injected with rAAV-CMVen-STRC mini promoter-Tprn-WPRE- SV40 virus showed significant hearing recovery.

FIG. 5 is a diagram showing the hearing restoration of a mouse with rAAV-CMVen-STRC mini promoter-Tprn-WPRE- SV40. (a) Schematic diagram of Tprn KO mice. Using Crispr Cas9 technology, the exon 1-4 region, approximately 7614 nt bases, was excised. The genotype of the Tprn KO mice can be identified using two pairs of primers: P1, P2 and P3, P4, for which the primers could be designed according to actual needs by those skilled in the art. (b) PCR gel electrophoresis image for identification of the Tprn KO mouse tail. WT: normal mice; KO: Tprn KO mice; -: ddH2O. (c) Hearing test threshold graph. Black: normal mice; red: Tprn KO mice; green: mice treated by injection of rAAV-CMVen-STRC mini promoter-Tprn-WPRE- SV40. (d) Hearing test waveform graph at 11.3 kHz. Black: normal mice, with a threshold of 15 dB; red: Tprn KO mice, with a threshold of 75 dB; green: mice treated by injection of rAAV-CMVen-STRC mini promoter-Tprn-WPRE- SV40, with a threshold of 35 dB. (e) confocal imaging of normal mice, Tprn KO mice, mice treated by injection of rAAV-CMVen-STRC mini promoter-Tprn-WPRE- SV40. The results showed that rAAV-CMVen-STRC mini promoter-Tprn-WPRE- SV40 can be used for the expression of TPRN protein in hair cells and the restoration of hearing in mice.

| | |
|---|---|
| P1: 5' | -GAGATTATGGCCCTTCAGAGTCAC-3' (SEQ ID NO: 35); |
| P2: 5' | -GAGTTGCAGAGCCCAGAGGTTAGG-3' (SEQ ID NO: 36); |
| P3: 5' | -GCTGCTTCCTTGATGCCTCCTACA-3' (SEQ ID NO: 37); |
| P4: 5' | -TGCTCAATGGCTGCTCCTCACTTC-3' (SEQ ID NO: 38). |

### 6. Regulation of gene expression with the promoter CAG

The constitutive promoter CAG can regulate the expression of fluorescent protein in the supporting cells, hair cells and spiral ganglion neurons, as well as in the supporting cells and hair cells of the vestibular system.

### a. Construction of viruses containing the promoter CAG

According to the construction method in a and b of 2 in Embodiment 1, rAAV-CAG-NLS-mNeonGreen-WPRE- SV40 virus was obtained only by removing the CMV enhancer fragment and replacing the STRC promoter with CAG.

b. The in vivo verification results of mice are shown below:
The operation method was the same as that in c of 2 in Embodiment 1.

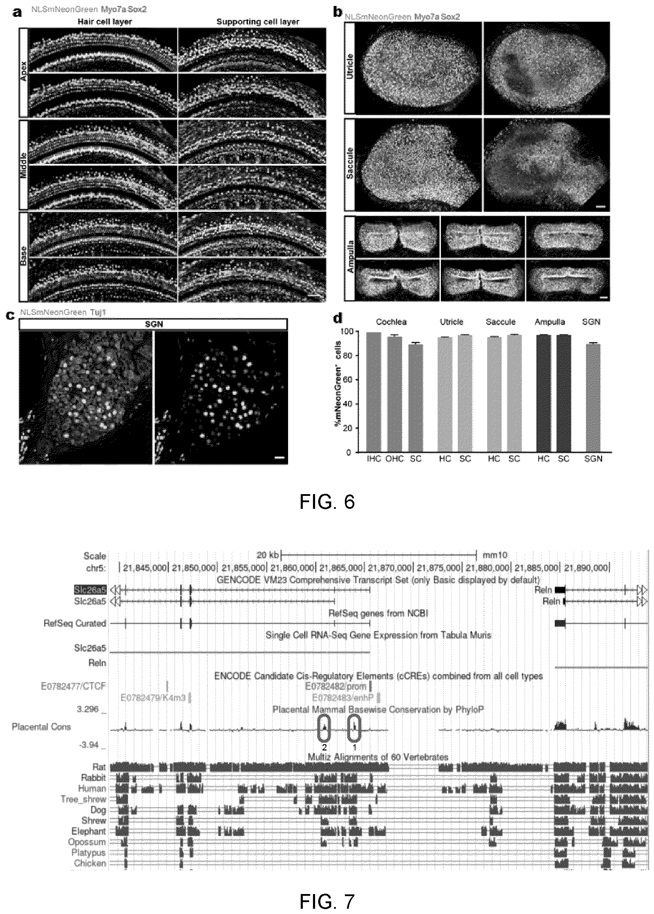

FIG. 6 shows schematic diagrams of cochlea (a), vestibule (b) and spiral ganglion neurons (c) of mice injected with rAAV-CAG-NLS-mNeonGreen-WPRE- SV40 virus. In FIGs. a and b, purple indicates a marker protein Myo7a of hair cells, red indicates a marker protein Sox2 of supporting cells, and green indicates virus-infected cells; in FIG. c, purple indicates a marker protein Tuj1 of spiral ganglion neurons, green indicates virus-infected cells, and d shows the infection efficiencies of different kinds of cochlear cells.

### Embodiment 2 Promoter Slc26a5

### 1. Isolation and identification of the promoter Slc26a5

### a. Synthesis of the promoter Slc26a5 sequence

In the Mouse GRCm39/mm39 genome provided according to http://www.genome.ucsc.edu/, Slc26a5 was compared with various species such as rats, guinea pigs, rabbits, etc., obtaining some highly conserved sequence regions in the transcription start region, as shown in FIG. 7. Two segments of sequence were combined by synthesis, and homologous arms were added at both the upstream and the downstream, in which the upstream homologous arm was: cacgcgttctcgattctaga (SEQ ID NO: 8) and the downstream homologous arm was: ctttctttctctctttcttt (SEQ ID NO: 9), to construct a vector of which the nucleotide sequence was shown as SEQ ID NO: 6, with the homologous arm part underlined. The promoter Slc26a5 sequence was synthesized by Shanghai Tsingke Biotechnology Co., Ltd.

SEQ ID NO: 6
cacgcgttctcgattctagaccatagcaaggagaggaaatgaaagtcaaaccagtttgcaa
cagaaagaaacttaagggtaaagaattcctaggtaaggctgataccgtaagtgtactgctggacagc
gctctggtctgttacgtaacaccgggagccccattctgtcgcacagaactgcctttttctctccttaactact
ctgaaactagacagaattacatggtcctcattaatttatgagtgataccaaaaggatcggctcaatccg
ggcttcagtgtatccaggcacaggactgtgctgtgcctcctctctactcccacaatgctttgctgcgttctc
agcttcccatctcctttgatgggtgaaactctcgcctatctgtgttgatcagctgttctgaaaggccccagg
agatgatagcatctcttctctgggtatccatgccagtgtttaatagcctcttgaaactgccgagaagtgctc
agcaattgtgtggtggggaaggacggtgtcagcaaggaataaagatgatctctcgcctcgacggtag
ctgaaatgtgagtttctgtgtccaggctcagataaaaagtgatcctcacacctttctcaagtagccagag
acgattgaatttctggaccagggcaggaaaacaagagcatgttgctaaccttgcttagtgctggggac
ctgatcttcttgccattacattgtcatttctatcattctcattcttgaaaaattatcctttgatttagagataactta
tgacaatgtgactctttatcttcctttctttctctctttcttt

### b. Amplification of the promoter Slc26a5

With the synthetic product DNA as the template, the promoter Slc26a5 was amplified by PCR using upstream and downstream primers. The amplification conditions for PCR were: predegeneration at 95°C for 5 min; 30 cycles of degeneration at 95°C for 30 s, annealing at 59°C for 30 s, and extension at 72°C for 1.5 min; extension at 72°C for 10 min. After confirmation of the PCR amplification product by gel electrophoresis with 1% agarose, the target fragments were recovered by gel slicing and sent to BioSune Technology (Shanghai, China) for sequencing. The results showed that: the amplified sequence was the Slc26a5 promoter sequence, with its nucleotide sequence shown as SEQ ID NO: 6. The recovered fragments were detected by Nanodrop2000 for the concentration.

### 2. Functional identification of the promoter Slc26a5

### a. Construction of a recombinant expression vector

First, an SHH mini promoter sequence was synthesized, and a homologous arm linked to the vector was added at the downstream of the sequence, in which the downstream homologous arm was: gctagcgaattcgccaccat (SEQ ID NO: 10), with its nucleotide sequence shown as SEQ ID NO: 7.

SEQ ID NO: 7
ctttctttctctctttctttcttttttaaactggaagccttcaggtgaaaataaagccacagcagcca
gaggggggagaaatgtagtctttctcagggttaacatcagaagacaagcttgtgcggcttggccaatc
agatgcgcccctgccagctataatatagtgcgaaaggcagcctgtctcacaagctctccagccttgcta
ccatttaaaatcaggctctttttgtcttttaattgctgtctcgagacccaactccgatgtgttccgttaccagc
gaccggcagcctgccatcgcagccccagtctgggtggggatcggagacaagtcccctgcagcagc
ggcaggcaaggttatataggaagagaaagagccaggcagcgccagagggaacgaacgagccg
agcgaggaagggagagccgagcgcaaggaggagcgcacacgcacacacccgcgcgtacccgc
tcgcgcacagacagcgcggggacagctcacaagtcctcaggttccgcggacgaggctagcgaattc
gccaccat

Using multi-fragment homologous recombination, the SHH mini promoter fragment and the Slc26a5 fragment were linked into pAAV-CAG-mNeonGreen-WPRE-SV40 plasmid digested with two enzymes, Xbal and Nhel, using a recombination kit (Vazyme, MultiS One Step Cloning Kit) by means of homologous recombination. The recombination system consists of: a linearized pAAV-CAG-mNeonGreen-WPRE-SV40 vector, 50 ng; clonal Slc26a5 promoter fragment, 30 ng; synthetic SHH mini promoter fragment, 30 ng; a recombinant ligase Exnase MultiS, 1 µL; a recombinant linking buffer 5x CE MultiS Buffer, 4 µL; ddH2O, supplement to 20 µL; reaction at 50°C for 20 min, to give the recombinant product.

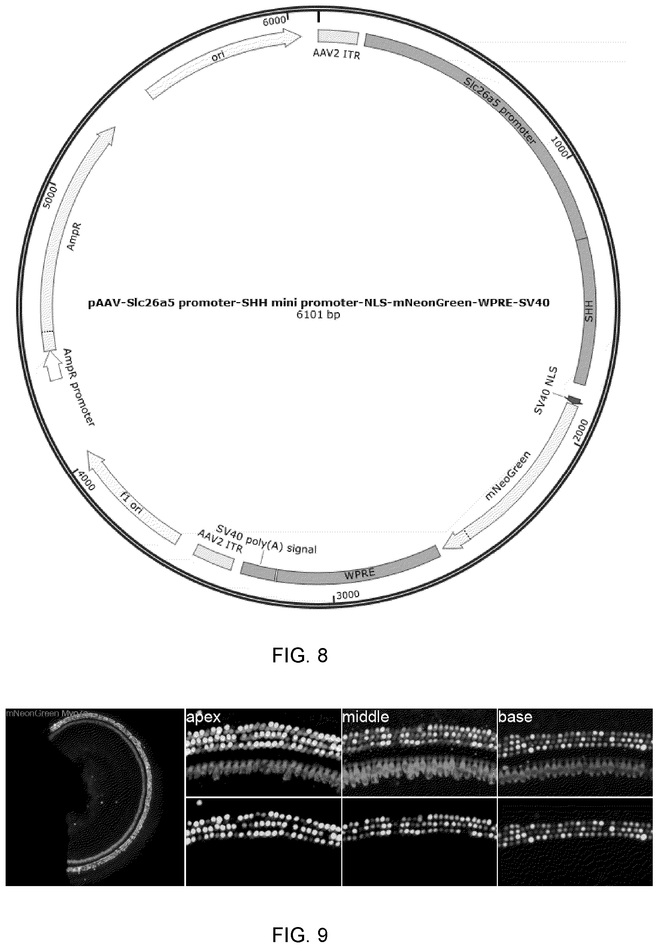

The ligation product was transformed into Escherichia Coli JM109 competent cells and placed in an incubator at 37°C overnight. Single colonies were picked for colony PCR identification. Positive colonies were subjected to expanded culture and plasmid extraction, followed by sequencing to verify the plasmid. The correctly sequenced plasmid was named as pAAV-Slc26a5 promoter-SHH mini promoter-NLS-mNeonGreen-WPRE-SV40. The diagram of the constructed plasmid recombinant expression vector is shown in FIG. 8.

### b. Virus production from a recombinant expression vector

The recombinant expression vector was virally packaged using the traditional triple plasmid packaging adeno-associated virus method as described in b of 2 in Embodiment 1, only with the plasmid recombinant expression vector being different, concentrated and purified by iodixanol gradient centrifugation, and finally detected by SYBR Green qPCR method for the titer of the recombinant adeno-associated virus. The titer of the finally obtained rAAV-Slc26a5 promoter-SHH mini promoter-NLS-mNeonGreen-WPRE-SV40 virus was 1×1013 vg/mL.

### 3. In vivo verification of the Slc26a5 promoter

The rAAV-Slc26a5 promoter-SHH mini promoter-NLS-mNeonGreen-WPRE-SV40 virus was injected into the cochlea of a 2-3-day-old C57BL/6J mouse through the round window. After the virus was fully expressed for 10-14 days, the mouse was executed by cervical dislocation. The injected cochlea was removed and placed in 4% paraformaldehyde, fixed at room temperature for 2 hours, washed with PBS three times, for 5 min each time, then decalcified with 0.5 mM EDTA, and soaked at room temperature for 2 hours to soften the cochlea. The basilar membrane was dissected under a stereo microscope with an ophthalmic scalpel into three parts: the apex, middle, and base, and the vestibular utricle tissue. By means of immunofluorescence staining, hair cells were labeled with anti-Myo7a antibodies and supporting cells were labeled with anti-Sox2 antibodies to prepare cochlea samples. The cochlea samples were imaged with a laser confocal scanning microscope, showing that virus-infected cells would express green fluorescent protein in their nuclei. The wavelength of the excitation light was 488 nm, 561 nm, 647 nm, respectively. The in vivo assay results are shown in FIG. 9, which shows a smear slide diagram of cochlea injected with rAAV-Slc26a5 promoter-SHH mini promoter-NLS-mNeonGreen-WPRE-SV40 virus, where purple indicates a marker protein Myo7a of hair cells, including the top three rows of outer hair cells and the bottom row of inner hair cells; green indicates virus-infected cells, indicating that the Slc26a5 promoter allows the fluorescent protein to be specifically expressed in the cochlear outer hair cells.

### Embodiment 3 Promoter otof

### 1. Isolation and identification of the promoter otof

### a. Design of the primer of the promoter otof

Based on the whole genome sequence of the C57BL/6J mouse strain provided by NCBI, an amplification primer was designed according to the sequence of the otof gene in mice, and according to the selected vector and the characteristics of the target gene, a homologous arm was added before the primer for the recombinant synthesis of a plasmid. The primer sequences are as below: upstream primer: cacgcgttctcgattctagagtactacagcccacagaagag (SEQ ID NO: 13), downstream primer: cttcccgctgctctggaactgccctttctttctctctttcttt (SEQ ID NO: 14). Both the upstream primer and the downstream primer carry homologous arm sequences, which are underlined respectively. The primer was synthesized by Shanghai GENEWIZ Biotechnology Co., Ltd.

### b. Amplification of the promoter otof

With the genomic DNA of the C57BL/6J mouse strain as the template, otof promoter fragments were amplified by PCR using upstream and downstream primers. The amplification conditions for PCR were: predegeneration at 95°C for 5 min; 30 cycles of degeneration at 95°C for 30 s, annealing at 59°C for 30 s, and extension at 72°C for 1.5 min; extension at 72°C for 10 min. After confirmation of the PCR amplification product by gel electrophoresis with 1% agarose, the target fragments were recovered by gel slicing and sent to BioSune Technology (Shanghai, China) for sequencing. The results showed that: the amplified sequence was the otof promoter sequence, with its nucleotide sequence shown as SEQ ID NO: 11, and the homologous arm was underlined. The recovered fragments were detected by Nanodrop2000 for the concentration.

SEQ ID NO: 11
cacgcgttctcgattctagagtactacagcccacagaagagcagtttatccttgtctaccatgt
gtgtcccagggatcaaactccagtcgccaggctgagccgtaggagcccttacctgctgagccatccc
accagcccaaagcctgaagactctcttagacaggcccctgagcttccaagccctgtcagtattagttgg
acatcttgccatgtcttgaacactagatgccctgtgaagatctcgagtgaaaagcatcagtgggggag
atccatgtagcaaccccaggccagtgatacctggatcaggagccactgaaagggggtcccaaagtc
cacctgagtgagggtgcagagtcacccctggggataagccgctgagaccaggaggcagggacaa
gatgggggacccctaccaagagtgagccgcaaaagcaacggcttagaatccagcccccagcactc
ctttccctacaagcccttcccgaaagagcagacttctgctgttccttggatatccctctacagatatccctct
gctacctctagccactgactagagcagggagcaagatggttgtcctctcactgaaaactactcatcaat
agtgtcgcctcctccagttctttgtctccagcctgggatgggtaggttggtgccctgactcttacaagtttcct
acctagcacagtagcccctgtgacctctcattagggcttccctagtcacagtctctcatttcaggaccag
ctgcctgaaattcaaaactatgacccaagaacagagcattagagcctagcatcccaagggcacaaa
cagtgcaggaccaggcatcaatgcattgtcacttctgatgctcctatataataggggccagtccttgggg
acctccttcaaactgccttatagaagctgctgctctccagactgtgtgtgtgtgtgtgtgtgtgtgtgtgtg
tgtgtgtgtgtgtgtgtgtatgtgtgtgtgtgtgtagtgtgtagatatacagatgtgtttatgtttatgtgtgga
agcctagggtcaacttcagttgtcttccttatttatttatctgacttgtttatttaaagacgggtctctttagcaa
acctggagcttctccattcagatagctccgctgccattaaactccaggggttctcctgtctccacctttctgc

caccgcagagctgggttagaagcccttgcccctgcaccggtctttaccatagactctggaatctaaact
cagatcttcatgctcctgtggcagacactttacctactgaggcatctcctttgccctcggctgaggcacag
catgatttatccagagcagtcaggtctgtcccgtccatgatctccaagccttctgagcatccacaaccct
ggtgattagctagacacatcaagagacatcgtggatactgcccaagagaccagtgccccattcaagc
tcagacagtgaagttccagatccccatgagaagtggcctgacccctcatctgcccatctgcatgccctc
tgcaatctgttggctctgagtggcacagcttgctctgccctgccaagtggcaggagcttccattccagga
gctggcaggagacacctgaagccggacagggctttatctcggagctggtgctgctgaataatggattc
agcatgcgttcctgggctcctgcacacactcactcacacacacacacacacacacacacacacaca
cacacacacgcgcgctacacacatgctgggctgacaacccagaggggcggggctgccggggctg
cacagctctgatgagctgaaggagcagcttgctttggttgccttggtctctgtgggcagcagcaggagg
aggcggcagcagccagagaagagggaggcgtgtgagccacactccaccagcgagcttcttcccgc
tgctctggaactgccctttctttctctctttcttt

### 2. Functional identification of the promoter otof

### a. Construction of a recombinant expression vector

First, an SHH mini promoter sequence was synthesized, and a homologous arm linked to the vector was added at the downstream of the sequence, in which the downstream homologous arm was: gctagcgaattcgccaccat (SEQ ID NO: 15). The homologous arm was underlined, and its nucleotide sequence was shown as SEQ ID NO: 12.

SEQ ID NO: 12
ctttctttctctctttctttcttttttaaactggaagccttcaggtgaaaataaagccacagcagcca
gaggggggagaaatgtagtctttctcagggttaacatcagaagacaagcttgtgcggcttggccaatc
agatgcgcccctgccagctataatatagtgcgaaaggcagcctgtctcacaagctctccagccttgcta
ccatttaaaatcaggctctttttgtcttttaattgctgtctcgagacccaactccgatgtgttccgttaccagc
gaccggcagcctgccatcgcagccccagtctgggtggggatcggagacaagtcccctgcagcagc
ggcaggcaaggttatataggaagagaaagagccaggcagcgccagagggaacgaacgagccg
agcgaggaagggagagccgagcgcaaggaggagcgcacacgcacacacccgcgcgtacccgc
tcgcgcacagacagcgcggggacagctcacaagtcctcaggttccgcggacgaggctagcgaattc

gccaccat

By means of multi-fragment homologous recombination, the SHH mini promoter fragment and the otof fragment were linked into pAAV-CAG-mNeonGreen-WPRE-SV40 plasmid digested with two enzymes, Xbal and Nhel, using a recombination kit (Vazyme, MultiS One Step Cloning Kit) by means of homologous recombination. The recombination system consists of: a linearized pAAV-CAG-mNeonGreen-WPRE-SV40 vector, 50 ng; clonal otof promoter fragment, 30 ng; synthetic SHH mini promoter fragment, 30 ng; a recombinant ligase Exnase MultiS, 1 µL; a recombinant linking buffer 5x CE MultiS Buffer, 4 µL; ddH2O, supplement to 20 µL; reaction at 50°C for 20 min, to give the recombinant product.

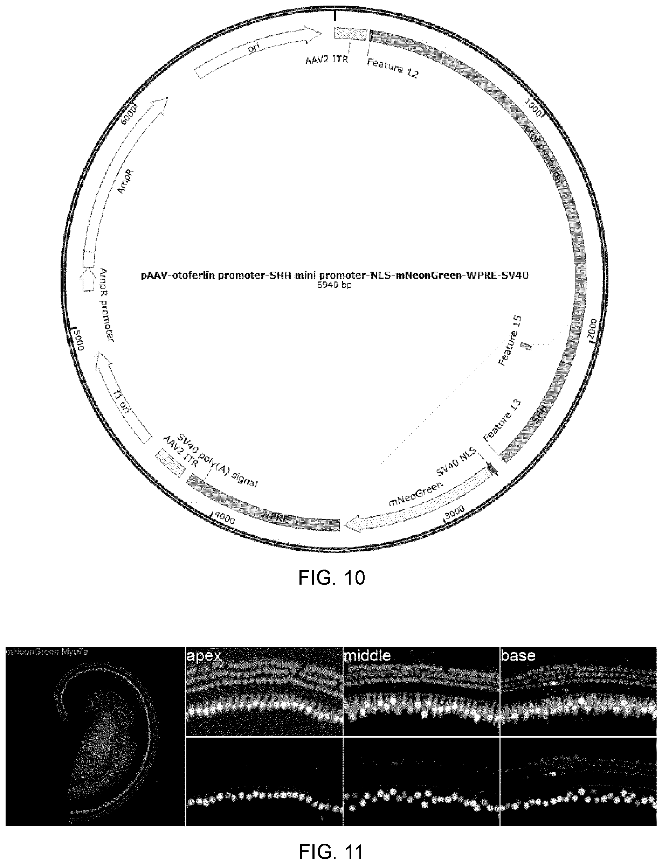

The ligation product was transformed into Escherichia Coli JM109 competent cells and placed in an incubator at 37°C overnight. Single colonies were picked for colony PCR identification. Positive colonies were subjected to expanded culture and plasmid extraction, followed by sequencing to verify the plasmid. The correctly sequenced plasmid was named as pAAV- otof promoter-SHH mini promoter-NLS-mNeonGreen-WPRE-SV40. The diagram of the constructed expression vector is shown in FIG. 10.

### b. Virus production from a recombinant expression vector

The recombinant expression vector was virally packaged using the traditional triple plasmid packaging adeno-associated virus method as described in b of 2 in Embodiment 1, only with the plasmid recombinant expression vector being different, concentrated and purified by iodixanol gradient centrifugation, and finally detected by SYBR Green qPCR method for the titer of the recombinant adeno-associated virus. The titer of the finally obtained rAAV-otof promoter-SHH mini promoter-NLS-mNeonGreen-WPRE-SV40 virus was 1×1013 vg/mL.

### 3. In vivo verification of the otof promoter

The rAAV-otof promoter-SHH mini promoter-NLS-mNeonGreen-WPRE-SV40 virus was injected into the cochlea of a 2-3 day-old C57BL/6J mouse through the round window. After the virus was fully expressed for 10-14 days, the mouse was executed by cervical dislocation. The injected cochlea was removed and placed in 4% paraformaldehyde, fixed at room temperature for 2 hours, washed with PBS three times, for 5 min each time, then decalcified with 0.5 mM EDTA, and soaked at room temperature for 2 hours to soften the cochlea. The basilar membrane was dissected under a stereo microscope with an ophthalmic scalpel into three parts: the apex, middle, and base. By means of immunofluorescence staining, hair cells were labeled with anti-Myo7a antibodies and supporting cells were labeled with anti-Sox2 antibodies to prepare cochlea samples. The cochlea samples were imaged with a laser confocal scanning microscope, showing that virus-infected cells would express green fluorescent protein in their nuclei. The wavelength of the excitation light was 488 nm, 561 nm, 647 nm, respectively. The in vivo assay results are shown in FIG. 11, which shows a smear slide diagram of cochlea injected with rAAV-otof promoter-SHH mini promoter-NLS-mNeonGreen-WPRE-SV40 virus, where purple indicates a marker protein Myo7a of hair cells, including the top three rows of outer hair cells and the bottom row of inner hair cells; green indicates virus-infected cells, indicating that the otof promoter allows the fluorescent protein to be specifically expressed in the cochlear inner hair cells.

### Embodiment 4 Promoter Sox10

### 1. Isolation and identification of the promoter Sox10

### a. Synthesis of the promoter Sox10 sequence

The promoter Sox10 sequence was derived from MCS7 in the Identification of neural crest and glial enhancers at the mouse Sox10 locus through transgenesis in zebrafish. G. Homologous arms were designed at both the upstream and the downstream of the sequence to construct a vector, in which the upstream homologous arm was: cacgcgttctcgattctaga (SEQ ID NO: 18) and the downstream homologous arm was: ctttctttctctctttcttt (SEQ ID NO: 19), with its nucleotide sequence shown as SEQ ID NO: 16 and the homologous arm part underlined. The sequence was synthesized by Shanghai Tsingke Biotechnology Co., Ltd.

SEQ ID NO: 16
cacgcgttctcgattctagagtggcctcaggcccctgtgcccgcgcccatggggttaaatctct
ccctgtctctctctgctccaagttgttttccaagcgaggcagagaatggttctcttgttaccaacatggcttct
gggcattgggtaatgcgttccctcttctccctgccctgcgccacaaagggaccttttgttcctctcccggcc
cctctctctggctcttctctgccccccacccaccccaagtccctcctccccacgcctcctgccctactctgc
gcgcacccctgggctgtgaggagtgaccgtggcctggctttctttctctctttcttt

### b. Amplification of the promoter Sox10

With the synthetic product DNA as the template, the promoter Sox10 was amplified by PCR using upstream and downstream primers. The amplification conditions for PCR were: predegeneration at 95°C for 5 min; 30 cycles of degeneration at 95°C for 30 s, annealing at 59°C for 30 s, and extension at 72°C for 1.5 min; extension at 72°C for 10 min. After confirmation of the PCR amplification product by gel electrophoresis with 1% agarose, the target fragments were recovered by gel slicing and sent to BioSune Technology (Shanghai, China) for sequencing. The results showed that: the amplified sequence was the Sox10 promoter sequence, with its nucleotide sequence shown as SEQ ID NO: 1. The recovered fragments were detected by Nanodrop2000 for the concentration.

### 2. Functional identification of the promoter Sox10

### a. Construction of a recombinant expression vector

First, an SHH mini promoter sequence was synthesized, and a homologous arm linked to the vector was added at the downstream of the sequence, in which the downstream homologous arm was: gctagcgaattcgccaccat (SEQ ID NO: 20). The sequence was synthesized by Shanghai Tsingke Biotechnology Co., Ltd., with its nucleotide sequence shown as SEQ ID NO: 17 and the homologous arm part underlined.

SEQ ID NO: 17
ctttctttctctctttctttcttttttaaactggaagccttcaggtgaaaataaagccacagcagcca
gaggggggagaaatgtagtctttctcagggttaacatcagaagacaagcttgtgcggcttggccaatc
agatgcgcccctgccagctataatatagtgcgaaaggcagcctgtctcacaagctctccagccttgcta
ccatttaaaatcaggctctttttgtcttttaattgctgtctcgagacccaactccgatgtgttccgttaccagc
gaccggcagcctgccatcgcagccccagtctgggtggggatcggagacaagtcccctgcagcagc
ggcaggcaaggttatataggaagagaaagagccaggcagcgccagagggaacgaacgagccg
agcgaggaagggagagccgagcgcaaggaggagcgcacacgcacacacccgcgcgtacccgc
tcgcgcacagacagcgcggggacagctcacaagtcctcaggttccgcggacgaggctagcgaattc
gccaccat

By means of multi-fragment homologous recombination, the SHH mini promoter fragment and the Sox10 fragment were linked into pAAV-CAG-mNeonGreen-WPRE-SV40 plasmid digested with two enzymes, Xbal and Nhel, using a recombination kit (Vazyme, MultiS One Step Cloning Kit) by means of homologous recombination. The recombination system consists of: a linearized pAAV-CAG-mNeonGreen-WPRE-SV40 vector, 50 ng; clonal Sox10 promoter fragment, 30 ng; synthetic SHH mini promoter fragment, 30 ng; a recombinant ligase Exnase MultiS, 1 µL; a recombinant linking buffer 5x CE MultiS Buffer, 4 µL; ddH2O, supplement to 20 µL; reaction at 50°C for 20 min, to give the recombinant product.

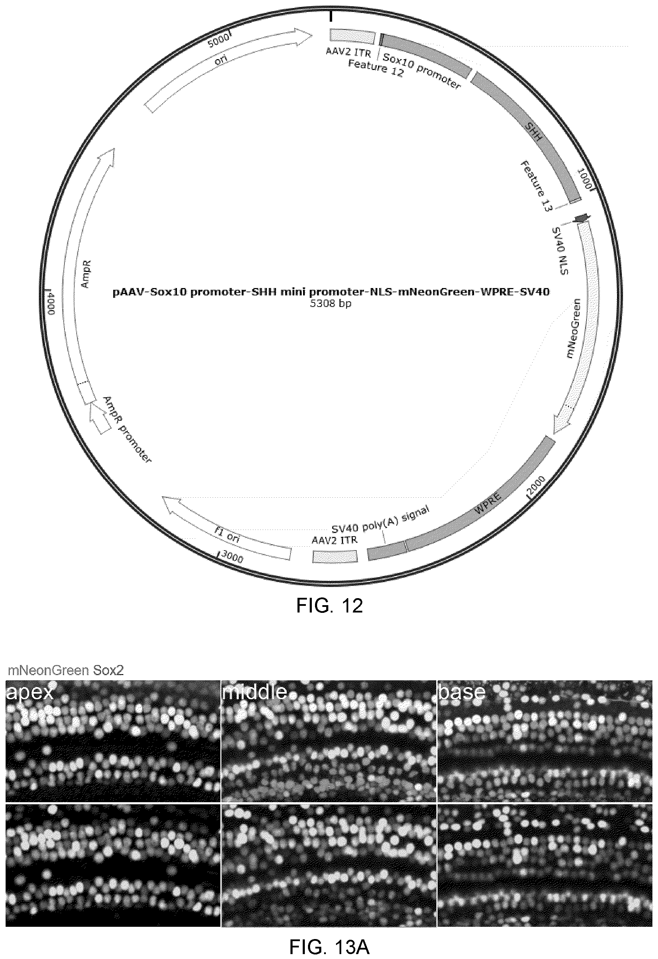

The ligation product was transformed into Escherichia Coli JM109 competent cells and placed in an incubator at 37°C overnight. Single colonies were picked for colony PCR identification. Positive colonies were subjected to expanded culture and plasmid extraction, followed by sequencing to verify the plasmid. The correctly sequenced plasmid was named as pAAV-Sox10 promoter-SHH mini promoter-NLS-mNeonGreen-WPRE-SV40. The diagram of the constructed plasmid recombinant expression vector is shown in FIG. 12.

### b. Virus production from a recombinant expression vector

The recombinant expression vector was virally packaged using the traditional triple plasmid packaging adeno-associated virus method as described in b of 2 in Embodiment 1, only with the plasmid recombinant expression vector being different, concentrated and purified by iodixanol gradient centrifugation, and finally detected by SYBR Green qPCR method for the titer of the recombinant adeno-associated virus. The titer of the finally obtained rAAV-Sox10 promoter-SHH mini promoter-NLS-mNeonGreen-WPRE-SV40 virus was 1×1013 vg/mL.

### 3. In vivo verification of the Sox10 promoter

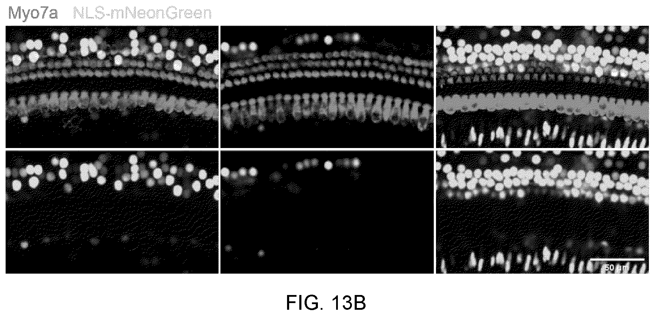

The rAAV-Sox10 promoter-SHH mini promoter-NLS-mNeonGreen-WPRE-SV40 virus was injected into the cochlea of a 2-3 day-old C57BL/6J mouse through the round window. After the virus was fully expressed for 10-14 days, the mouse was executed by cervical dislocation. The injected cochlea was removed and placed in 4% paraformaldehyde, fixed at room temperature for 2 hours, washed with PBS three times, for 5 min each time, then decalcified with 0.5 mM EDTA, and soaked at room temperature for 2 hours to soften the cochlea. The basilar membrane was dissected under a stereo microscope with an ophthalmic scalpel into three parts: the apex, middle, and base. By means of immunofluorescence staining, hair cells were labeled with anti-Myo7a antibodies and supporting cells were labeled with anti-Sox2 antibodies to prepare cochlea samples. The cochlea samples were imaged with a laser confocal scanning microscope, showing that virus-infected cells would express green fluorescent protein in their nuclei. The wavelength of the excitation light was 488 nm, 561 nm, 647 nm, respectively. The in vivo assay results are shown in FIG. 13, which shows a smear slide diagram of cochlea injected with rAAV-Sox10 promoter-SHH mini promoter-NLS-mNeonGreen-WPRE-SV40 virus, where red a marker protein Sox2 of supporting cells, purple indicates a marker protein Myo7a of hair cells, green indicates virus-infected cells, indicating that the Sox10 promoter allows the fluorescent protein to be specifically expressed in the cochlear supporting cells.

### Embodiment 5 Promoter Ngfr 1

### 1. Isolation and identification of the promoter Ngfr 1

### a. Synthesis of the promoter Ngfr 1 sequence

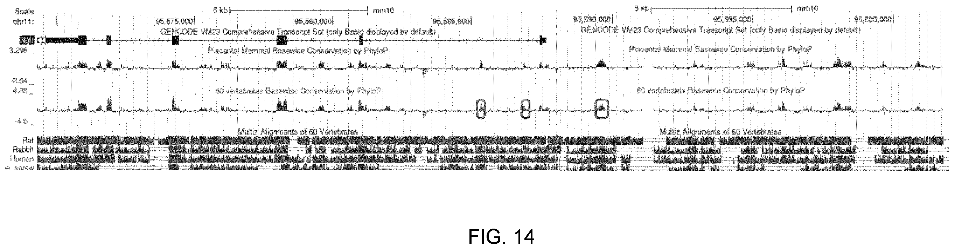

In the Mouse GRCm39/mm39 genome provided according to http://www.genome.ucsc.edu/, Ngfr was compared with various species such as rats, guinea pigs, rabbits, etc., obtaining some highly conserved sequence regions in the transcription start region, as shown in FIG. 14. Three segments of sequence were combined by synthesis, and homologous arms were added at both ends, in which the upstream homologous arm was: cacgcgttctcgattctaga (SEQ ID NO: 23) and the downstream homologous arm was: ctttctttctctctttcttt (SEQ ID NO: 24), to construct a vector of which the nucleotide sequence was shown as SEQ ID NO: 21, with the homologous arm part underlined. The promoter Ngfr 1 sequence was synthesized by Shanghai Tsingke Biotechnology Co., Ltd.

SEQ ID NO: 21

cacgcgttctcgattctagagatccgaagcatcttatgctcagcataaccggaggtgccctgg

gtacgtggggcagcccactgagaagccacagcggggcacacgggtgcggctcctctcacgcctca
cttgactgctctggactccccaccctaggcagccgggcgggcgcggttccggaggggttgggggggg
gggggagttgggcgggctgggcggggaggaggcggggctgctgcattgccttcacccagctgctc
ccgcccgctagcagccagagcgagtggagcctccgccagctccggcgggcagcgggcgctggag
cgcagcgcagcgcagctcagctcagcgcgcagcaccatcggtccgcagagcggactgagctaga
agctgagcgctgtcgcagactgggagcagtctcgagtggggtggagggaggtttgggaatctcgggc
gcctgggttcttttgacgacagcgtgtgaattttgggatcccgaggggagttttctctttagcatcttagtccc
tggggagttgtagggcagagagaaggaagggaaagagatcggcactaaagatgtgaggggctag
agacggaacaatgcgggaggcagctggagggactggaggcagaggggacccagtatggacaat
ggggaccggccagacaatgggggggaggggcatcgaaaggagcgattcgggatagagccggttc
tgtgttccgccgcggaccgagcctcttcctgcgccgctgcgagagttctgaaaccagagcgcaacca
gagtctgctggccggccgcgccggtaatggaggcactttgtcattcagacgtctgtaaccggagccgc
ccggctggctaatgctcctaatagggatgcagcagcgtaggcaacaaatgggcagcatgagcgtcct
ttctttctctctttcttt

### b. Amplification of the promoter Ngfr 1

With the synthetic product DNA as the template, the promoter Ngfr 1 was amplified by PCR using upstream and downstream primers. The amplification conditions for PCR were: predegeneration at 95°C for 5 min; 30 cycles of degeneration at 95°C for 30 s, annealing at 59°C for 30 s, and extension at 72°C for 1.5 min; extension at 72°C for 10 min. After confirmation of the PCR amplification product by gel electrophoresis with 1% agarose, the target fragments were recovered by gel slicing and sent to BioSune Technology (Shanghai, China) for sequencing. The results showed that: the amplified sequence was the Ngfr 1 promoter sequence, with its nucleotide sequence shown as SEQ ID NO: 1. The recovered fragments were detected by Nanodrop2000 for the concentration.

### 2. Functional identification of the promoter Ngfr 1

### a. Construction of a recombinant expression vector

First, an SHH mini promoter sequence was synthesized, and a homologous arm linked to the vector was added at the downstream of the sequence, in which the downstream homologous arm was: gctagcgaattcgccaccat (SEQ ID NO: 25), with its nucleotide sequence shown as SEQ ID NO: 22 and the homologous arm part underlined.

SEQ ID NO: 22
ctttctttctctctttctttcttttttaaactggaagccttcaggtgaaaataaagccacagcagcca
gaggggggagaaatgtagtctttctcagggttaacatcagaagacaagcttgtgcggcttggccaatc
agatgcgcccctgccagctataatatagtgcgaaaggcagcctgtctcacaagctctccagccttgcta
ccatttaaaatcaggctctttttgtcttttaattgctgtctcgagacccaactccgatgtgttccgttaccagc
gaccggcagcctgccatcgcagccccagtctgggtggggatcggagacaagtcccctgcagcagc
ggcaggcaaggttatataggaagagaaagagccaggcagcgccagagggaacgaacgagccg
agcgaggaagggagagccgagcgcaaggaggagcgcacacgcacacacccgcgcgtacccgc
tcgcgcacagacagcgcggggacagctcacaagtcctcaggttccgcggacgaggctagcgaattc
gccaccat

By means of multi-fragment homologous recombination, the SHH mini promoter fragment and the Ngfr 1 fragment were linked into pAAV-CAG-mNeonGreen-WPRE-SV40 plasmid digested with two enzymes, Xbal and Nhel, using a recombination kit (Vazyme, MultiS One Step Cloning Kit) by means of homologous recombination. The recombination system consists of: a linearized pAAV-CAG-mNeonGreen-WPRE-SV40 vector, 50 ng; clonal Ngfr 1 promoter fragment, 30 ng; synthetic SHH mini promoter fragment, 30 ng; a recombinant ligase Exnase MultiS, 1 µL; a recombinant linking buffer 5x CE MultiS Buffer, 4 µL; ddH2O, supplement to 20 µL; reaction at 50°C for 20 min, to give the recombinant product.

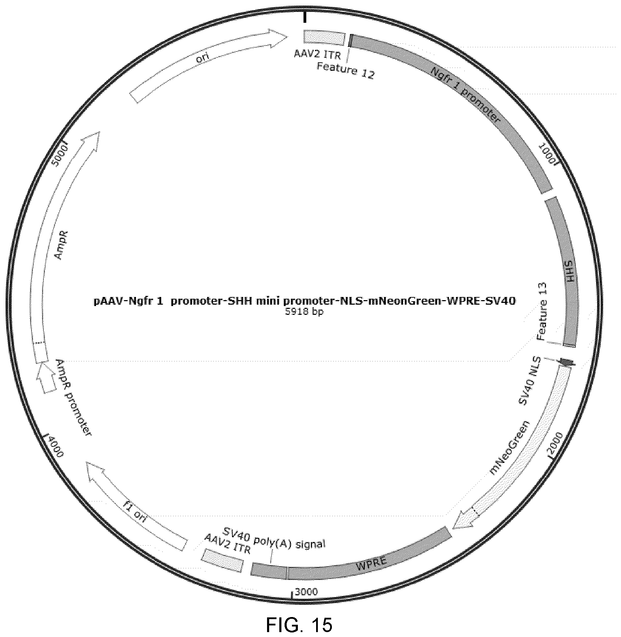

The ligation product was transformed into Escherichia Coli JM109 competent cells and placed in an incubator at 37°C overnight. Single colonies were picked for colony PCR identification. Positive colonies were subjected to expanded culture and plasmid extraction, followed by sequencing to verify the plasmid. The correctly sequenced plasmid was named as pAAV- Ngfr 1 promoter-SHH mini promoter-NLS-mNeonGreen-WPRE-SV40. The diagram of the constructed plasmid recombinant expression vector is shown in FIG. 15.

### b. Virus production from a recombinant expression vector

The recombinant expression vector was virally packaged using the traditional triple plasmid packaging adeno-associated virus method as described in b of 2 in Embodiment 1, only with the plasmid recombinant expression vector being different, concentrated and purified by iodixanol gradient centrifugation, and finally detected by SYBR Green qPCR method for the titer of the recombinant adeno-associated virus. The titer of the finally obtained rAAV- Ngfr 1 promoter-SHH mini promoter-NLS-mNeonGreen-WPRE-SV40 virus was 1×1013 vg/mL.

### 3. In vivo verification of the Ngfr 1 promoter

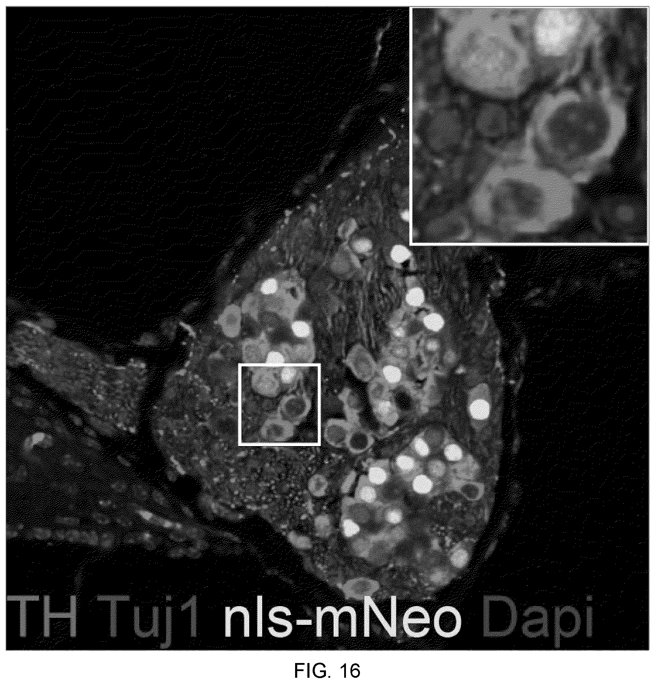

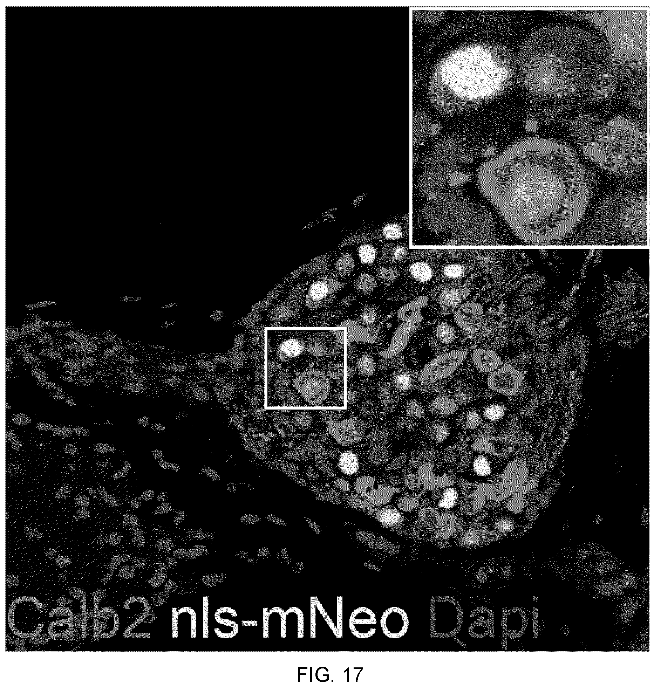

The rAAV- Ngfr 1 promoter-SHH mini promoter-NLS-mNeonGreen-WPRE-SV40 virus was injected into the cochlea of a 2-3-day-old C57BL/6J mouse through the round window. After the virus was fully expressed for 10-14 days, the mouse was executed by cervical dislocation. The injected cochlea was removed and placed in 4% paraformaldehyde, fixed at room temperature for 2 hours, washed with PBS three times, for 5 min each time, then decalcified with 0.5 mM EDTA, and soaked at room temperature for 2 hours to soften the cochlea. The softened cochlea was then dehydrated overnight with gradient sucrose and sliced into 0.2 mm samples using a freezing microtome. By means of immunofluorescence staining, spiral ganglion neurons were labeled with anti-Tuj1 antibodies, type I spiral ganglion neurons were labeled with anti-Calb2 antibodies and type II spiral ganglion neurons were labeled with anti-TH antibodies to prepare cochlea samples. The cochlea samples were imaged with a laser confocal scanning microscope, showing that virus-infected cells would express green fluorescent protein in their nuclei. The wavelength of the excitation light was 488 nm, 561 nm, 647 nm, respectively. The in vivo assay results are shown in FIGs. 16 and 17.

FIG. 16 shows that the Ngfr 1 promoter can specifically initiate the expression of genes in the SGNs, but not in type II spiral ganglion neurons. This figure shows a slice diagram of cochlea injected with rAAV- Ngfr 1 promoter-SHH mini promoter-NLS-mNeonGreen-WPRE-SV40 virus, in which purple indicates a marker protein Tuj1 of spiral ganglion neurons, red indicates a marker protein TH of type II spiral ganglion neurons, green indicates virus-infected cells, indicating that the Ngfr 1 promoter does not specifically initiate the expression of the fluorescent protein in the type II spiral ganglion neurons.

FIG. 17 shows that the Ngfr 1 promoter can specifically initiate the expression of genes in type I spiral ganglion neurons. This figure shows a slice diagram of cochlea injected with rAAV- Ngfr 1 promoter-SHH mini promoter-NLS-mNeonGreen-WPRE-SV40 virus, in which red indicates a marker protein Calb2 of type I spiral ganglion neurons, green indicates virus-infected cells, indicating that the Ngfr 1 promoter specifically initiates the expression of the fluorescent protein in the type I spiral ganglion neurons.

### Embodiment 6 Promoter Ngfr 2

### 1. Isolation and identification of the promoter Ngfr 2

### a. Synthesis of the promoter Ngfr 2 sequence

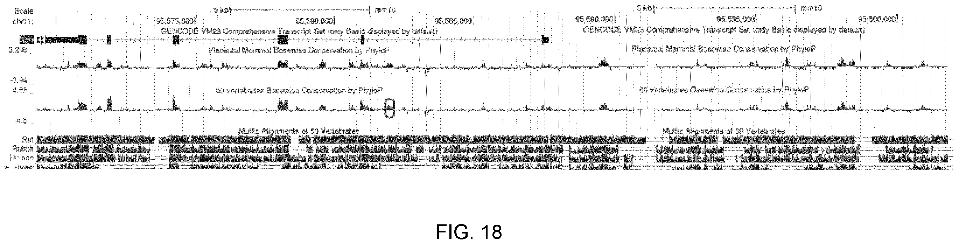

In the Mouse GRCm39/mm39 genome provided according to http://www.genome.ucsc.edu/, Ngfr was compared with various species such as rats, guinea pigs, rabbits, etc., obtaining some highly conserved sequence regions in the transcription start region, as shown in FIG. 18. The sequences were combined by synthesis, and homologous arms were added at both ends, in which the upstream homologous arm was: cacgcgttctcgattctaga (SEQ ID NO: 28) and the downstream homologous arm was: ctttctttctctctttcttt (SEQ ID NO: 29), to construct a vector of which the nucleotide sequence was shown as SEQ ID NO: 26. The sequence was synthesized by Shanghai Tsingke Biotechnology Co., Ltd.

SEQ ID NO: 26
cacgcgttctcgattctagagccgagctctatgtgatgaaaggaaatcagaacagacccttct
gaaatctcagctcctgggagaagcctgcctcaagtacctcagggctcctggtctgcagatgttgccatt
gccagccagcactcccactgccagcacctgggaggagatggtggcagcttccggtgctgcctggact
ctctctgctgcccttatggagaccactctgcccctagggcatcacccagtgttttctcctgcctctgtttacttt
tctgtcaaactctggtgaaaccacagtggtgtgtgtgtgtgtgtgtgtgtgtgtgtgtggtgtgtcttgg
caaagatcggtccagcatggaagcaggagaggaatgcccaaacaatcttgtaaagctctctggtctc
acgaccaaagcccaaattgctcagcatcaaatggggtgggtggggggtgtgtgcgcaaggtctgga
gcctgcagagaaggctttggagttttattagacattaagattgttctttaaggaacagaaacaaagtgag
cctccaacagcccctccctcgggggaatctggtgctaagtcgttccatccatgagcaaagatgctttagt
ggcaggaatctgactccccccccccccatccagctctgttcccaggggccacatgggaggagaggg
gaggggatggagtcaggctaggacggggaagtcaactctggaagaatagagttgctggctggtgaa
agccggccactgataacaggcgtccctttctctgtcttggctccagacttgcccatcagtctttgccctggc
accaagctgtccctgtgtttatctgcagaggggtgaaaactcccaaaggaaacccagggcaggcca
gacacctccagtacagagtcaatatttgaacccaagtcctcactgccttctgttcttggacatagacaact
ccgttttgatatttaatatgtggaatgagacctggagaagggccctgttctcctttttaggaaagggccctg
cctctgggtaactgcagccctgcaggtgtgaagtttagcaaatggagtttgggccaaatttcagttccact
ggcccccttggctaggagccctgtgcaagctgcaacctgcccagccattctggatatcggtctcaccct
cctcctgtggtgtatcctcaggactccaggagaggctgtaaccctaaggtgaactctgcagaaggttag
ccactgaggctatgacctcacaccccagcagcagtcaagggccccactccccccacgccccccact
gccccccagccttccttcctgccagctttgatcctcctaatgcctcccgggtgagctcatcccagcccag

gtggttctaatagaaagtattaagagggaggcgtttgatcccttggaagacaccacctttgctcctgcca
ggctgaccttgtgatctccttccacctaggctttctttctctctttcttt

### b. Amplification of the promoter Ngfr 2

With the synthetic product DNA as the template, the promoter Ngfr 2 was amplified by PCR using upstream and downstream primers. The amplification conditions for PCR were: predegeneration at 95°C for 5 min; 30 cycles of degeneration at 95°C for 30 s, annealing at 59°C for 30 s, and extension at 72°C for 1.5 min; extension at 72°C for 10 min. After confirmation of the PCR amplification product by gel electrophoresis with 1% agarose, the target fragments were recovered by gel slicing and sent to BioSune Technology (Shanghai, China) for sequencing. The results showed that: the amplified sequence was the Ngfr 2 promoter sequence, with its nucleotide sequence shown as SEQ ID NO: 26. The recovered fragments were detected by Nanodrop2000 for the concentration.

### 2. Functional identification of the promoter Ngfr 2

### a. Construction of a recombinant expression vector

First, an SHH mini promoter sequence was synthesized, and a homologous arm linked to the vector was added at the downstream of the sequence, in which the downstream homologous arm was: gctagcgaattcgccaccat (SEQ ID NO: 30), with its nucleotide sequence shown as SEQ ID NO: 27 and the homologous arm underlined.

SEQ ID NO: 27
ctttctttctctctttctttcttttttaaactggaagccttcaggtgaaaataaagccacagcagcca
gaggggggagaaatgtagtctttctcagggttaacatcagaagacaagcttgtgcggcttggccaatc
agatgcgcccctgccagctataatatagtgcgaaaggcagcctgtctcacaagctctccagccttgcta
ccatttaaaatcaggctctttttgtcttttaattgctgtctcgagacccaactccgatgtgttccgttaccagc
gaccggcagcctgccatcgcagccccagtctgggtggggatcggagacaagtcccctgcagcagc
ggcaggcaaggttatataggaagagaaagagccaggcagcgccagagggaacgaacgagccg

agcgaggaagggagagccgagcgcaaggaggagcgcacacgcacacacccgcgcgtacccgc
tcgcgcacagacagcgcggggacagctcacaagtcctcaggttccgcggacgaggctagcgaattc
gccaccat

By means of multi-fragment homologous recombination, the SHH mini promoter fragment and the Ngfr 2 fragment were linked into pAAV-CAG-mNeonGreen-WPRE-SV40 plasmid digested with two enzymes, Xbal and Nhel, using a recombination kit (Vazyme, MultiS One Step Cloning Kit) by means of homologous recombination. The recombination system consists of: a linearized pAAV-CAG-mNeonGreen-WPRE-SV40 vector, 50 ng; clonal Ngfr 2 promoter fragment, 30 ng; synthetic SHH mini promoter fragment, 30 ng; a recombinant ligase Exnase MultiS, 1 µL; a recombinant linking buffer 5x CE MultiS Buffer, 4 µL; ddH2O, supplement to 20 µL; reaction at 50°C for 20 min, to give the recombinant product.

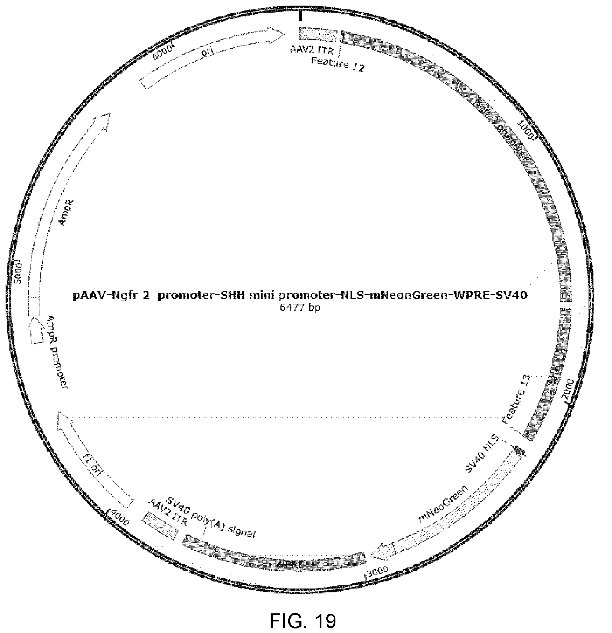

The ligation product was transformed into Escherichia Coli JM109 competent cells and placed in an incubator at 37°C overnight. Single colonies were picked for colony PCR identification. Positive colonies were subjected to expanded culture and plasmid extraction, followed by sequencing to verify the plasmid. The correctly sequenced plasmid was named as pAAV- Ngfr 2 promoter-SHH mini promoter-NLS-mNeonGreen-WPRE-SV40. The diagram of the constructed plasmid recombinant expression vector is shown in FIG. 19.

### b. Virus production from a recombinant expression vector

The recombinant expression vector was virally packaged using the traditional triple plasmid packaging adeno-associated virus method as described in b of 2 in Embodiment 1, only with the plasmid recombinant expression vector being different, concentrated and purified by iodixanol gradient centrifugation, and finally detected by SYBR Green qPCR method for the titer of the recombinant adeno-associated virus. The titer of the finally obtained rAAV-Ngfr 2 promoter-SHH mini promoter-NLS-mNeonGreen-WPRE-SV40 virus was 1×1013 vg/mL.

### 3. In vivo verification of the Ngfr 2 promoter

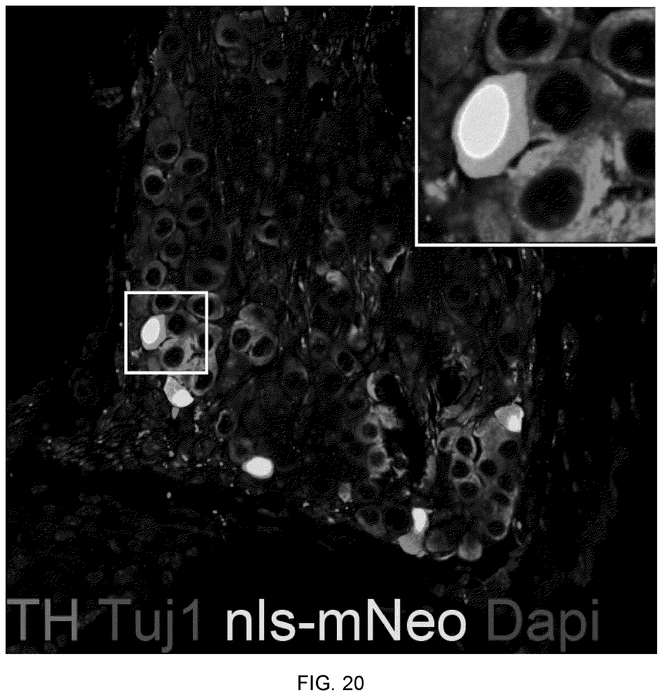

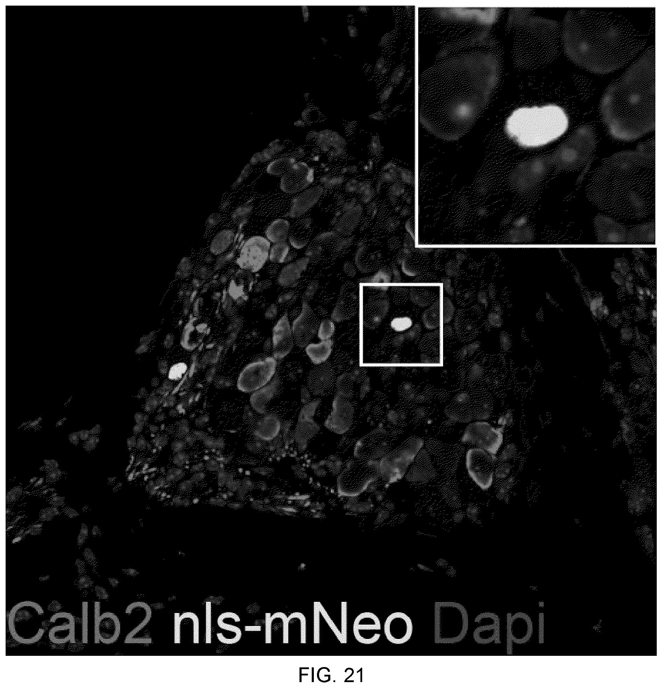

The rAAV-Ngfr 2 promoter-SHH mini promoter-NLS-mNeonGreen-WPRE-SV40 virus was injected into the cochlea of a 2-3-day-old C57BL/6J mouse through the round window. After the virus was fully expressed for 10-14 days, the mouse was executed by cervical dislocation. The injected cochlea was removed and placed in 4% paraformaldehyde, fixed at room temperature for 2 hours, washed with PBS three times, for 5 min each time, then decalcified with 0.5 mM EDTA, and soaked at room temperature for 2 hours to soften the cochlea. The softened cochlea was then dehydrated overnight with gradient sucrose and sliced into 0.2 mm samples using a freezing microtome. By means of immunofluorescence staining, spiral ganglion neurons were labeled with anti-Tuj1 antibodies, type I spiral ganglion neurons were labeled with anti-Calb2 antibodies and type II spiral ganglion neurons were labeled with anti-TH antibodies to prepare cochlea samples. The cochlea samples were imaged with a laser confocal scanning microscope, showing that virus-infected cells would express green fluorescent protein in their nuclei. The wavelength of the excitation light was 488 nm, 561 nm, 647 nm, respectively. The in vivo assay results are shown in FIGs. 20 and 21.

FIG. 20 shows that the Ngfr 2 promoter can specifically initiate the expression of genes in SGNs and type II spiral ganglion neurons. This figure shows a slice diagram of cochlea injected with rAAV-Ngfr 2 promoter-SHH mini promoter-NLS-mNeonGreen-WPRE-SV40 virus, in which purple indicates a marker protein Tuj1 of spiral ganglion neurons, red indicates a marker protein TH of type II spiral ganglion neurons, green indicates virus-infected cells, indicating that the Ngfr 1 promoter allows the fluorescent protein to be specifically expressed in the type II spiral ganglion neurons.

FIG. 21 shows that the Ngfr 2 promoter cannot specifically initiate the expression of genes in type I spiral ganglion neurons. This figure shows a slice diagram of cochlea injected with rAAV- Ngfr 2 promoter-SHH mini promoter-NLS-mNeonGreen-WPRE-SV40 virus, in which red indicates a marker protein Calb2 of type I spiral ganglion neurons, green indicates virus-infected cells, indicating that the Ngfr 2 promoter does not allow the fluorescent protein to be specifically expressed in the type I spiral ganglion neurons.

### Embodiment 7 Promoter Dnah5

### 1. Isolation and identification of the promoter Dnah5

### a. Design of the primer of the promoter Dnah5

Based on the whole genome sequence of the C57BL/6J mouse strain provided by NCBI, a primer was designed at the upstream of the transcriptional start site of the Dnah5 gene in mice, and homologous arms were added at both ends, in which the upstream primer was: ctattaccatggtgctcgaggtcacatgttgacaatgttctcgg (SEQ ID NO: 33), and the downstream primer was: gccatcccagattgctgggggtacctcgacggtaagtcc (SEQ ID NO: 34), to construct a vector, in which the underlines indicated the homologous arms.

### b. Amplification of the promoter Dnah5

With the C57BL/6J mice DNA as the template, the promoter Dnah5 was amplified by PCR using upstream and downstream primers. The amplification conditions for PCR were: predegeneration at 95°C for 5 min; 30 cycles of degeneration at 95°C for 30 s, annealing at 59°C for 30 s, and extension at 72°C for 1.5 min; extension at 72°C for 10 min. After confirmation of the PCR amplification product by gel electrophoresis with 1% agarose, the target fragments were recovered by gel slicing and sent to BioSune Technology (Shanghai, China) for sequencing. The results showed that: the amplified sequence was the Dnah5 promoter sequence, with its nucleotide sequence shown as SEQ ID NO: 31. The recovered fragments were detected by Nanodrop2000 for the concentration.

SEQ ID NO: 31
gtcacatgttgacaatgttctcgggactaatctgaactgcttagcagggacgatcagactatct
tgtcttattctgtgtctctcctgtgacctgtggccaccgttcacaattataacaacaaacagctgcttaccaa
tacacagaggttgattgaaaattatctttatatctcacgtatagtgtgcgcttcatttgcctgaggctctatgtt
aagtctgccgtgaccccggatgtattcacacaccatgttggctcagaagatgacactcccaaatgtac
agctgtgaagtttagagtgagaacccgtgaagagccgcacattcagggagagttttctttctccttatcta
tcttcaagtccacatagtccaggagacattatttgctgcaagtgctttcctcagccatcccagcaggaag
aggaaacacatgcaaatggcaggcaggagtgatggctaccacaggggcattaccccaaggcattg
tctgttctttgagtcaattcatctccgtgaaaaataatctactcttccgataaaattgctcaatacttttttttctct
cctcaaagaagaacagatgtaagcttctaaatcttactgaactgctgggcacctgggtgattcccgtgtg
tgcaatactctgcatggcttctcccttgtcaatctgtctactgcctttgtcagattcaaacactgaacttccaa
gggaaaatagagagttcttctgtgcctatacttctgactttaaatatgcttttgttatttgcttgccatcagtatt
caacaccaaatatctgtatccatattaacactacaaatggaggccacaccctaactggctgatggcta
ggtgaattgattgtcctgatatcaaggtagtgccctggttgtcaattctgtttctgatgaggttgaatagcag
gggactcctcaatttcctgatgatctttgaataatccgaaaaggatttatttactagattttattttacgttttaat
ttttttgagatagagtttctctatgtagctgtagccatctggaactcctctgtaaaccaggctagcctggaac
tcacagatatcggcctctatctcctgagtgctgggattaaaggtgagcaccactgctgcctagctaactg
ctggatttttaatcccaaggatccaatgaaatggaccttaaattctgccatttgtgtgctttgatgggagggt
tgtaggtgagaggtctctccccatccgtcggtgcggagaggaagatctagatgtttaggcttatggaatg
caatgctttgctttccatgcttgggcggccgtgtgaacaggagtaatttatggacaccatttgccatttgcat
atgaaataggctgcttgttctttatgggagaggaaatggctcctttgggtcacaattagcctatcaagcaa
ttagtcacaattagtcaccatgctggcagttaattgtgcacacaaagttgtttagacaaagtaagactga
atttcctaactcgagtaatatatccagagctgtgacttgtctgccttctgctttgatcaggtttaaggtttcttat

tgttcagagaaataatttaagctcctttattgactcaaagcttattgttgggcagtgtatggaatggaacatt
agaacttgtggttgcaaccaagcagttaaattgctttctcttgttgctaaacatgattaagaggcgtgctta
aacggcacagcatggaaacagtattaccctaacaacgaaggaccacagaatcgaggaaaggccc
cagggcagagactcagggaaggatatatgagcatcgcagatggactgacttctcgcctcagttctgtg
aatatcgagagtcacccacatgcatgagaagcaacagcgcgatgctgtgaccctcttagagtctttgc
ctgtgatattccactggaggatgaacagctctcttggtagttagaagaccattctggaggagctgacttg
gggccatcccagattgctggg

### 2. Functional identification of the promoter Dnah5

### a. Construction of a recombinant expression vector

First, a CMV enhancer sequence was synthesized, and a homologous arm linked to the vector was added at the upstream of the sequence. The homologous arm was underlined, and its nucleotide sequence was shown as SEQ ID NO: 32.

SEQ ID NO: 32
gttcctgcggccgcacgcgtgacattgattattgactagttattaatagtaatcaattacggggt
cattagttcatagcccatatatggagttccgcgttacataacttacggtaaatggcccgcctggctgacc
gcccaacgacccccgcccattgacgtcaataatgacgtatgttcccatagtaacgccaatagggacttt
ccattgacgtcaatgggtggactatttacggtaaactgcccacttggcagtacatcaagtgtatcatatgc
caagtacgccccctattgacgtcaatgacggtaaatggcccgcctggcattatgcccagtacatgacct
tatgggactttcctacttggcagtacatctacgtattagtcatcgctattaccatggtgctcgag

By means of multi-fragment homologous recombination, the promoter CMV enhancer fragment and the Dnah5 fragment were linked into pAAV-CAG-mNeonGreen-WPRE-SV40 plasmid digested with two enzymes, Kpn1 and Mlu1, using a recombination kit (Vazyme, MultiS One Step Cloning Kit) by means of homologous recombination. The recombination system consists of: a linearized pAAV-CAG-mNeonGreen-WPRE-SV40 vector, 50 ng; clonal Dnah5 promoter fragment, 30 ng; synthetic CMV enhancer fragment, 30 ng; a recombinant ligase Exnase MultiS, 1 µL; a recombinant linking buffer 5x CE MultiS Buffer, 4 µL; ddH2O, supplement to 20 µL; reaction at 50°C for 20 min, to give the recombinant product.

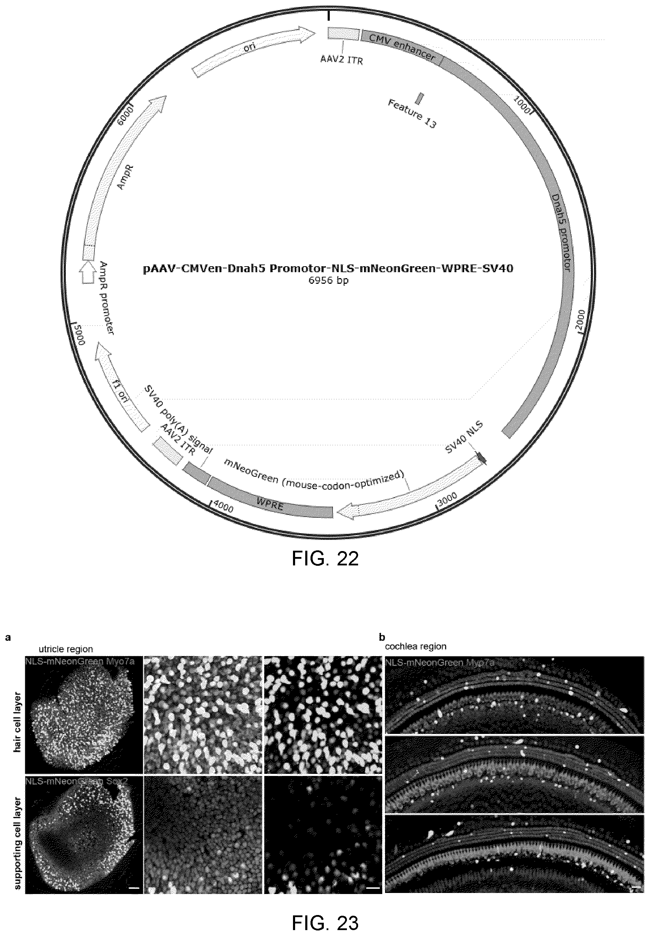

The ligation product was transformed into Escherichia Coli JM109 competent cells and placed in an incubator at 37°C overnight. Single colonies were picked for colony PCR identification. Positive colonies were subjected to expanded culture and plasmid extraction, followed by sequencing to verify the plasmid. The correctly sequenced plasmid was named as pAAV-CMVen-Dnah5 promoter-NLS-mNeonGreen-WPRE-SV40. The diagram of the constructed expression vector is shown in FIG. 22.

### b. Virus production from a recombinant expression vector

The recombinant expression vector was virally packaged using the traditional triple plasmid packaging adeno-associated virus method as described in b of 2 in Embodiment 1, only with the plasmid recombinant expression vector being different, concentrated and purified by iodixanol gradient centrifugation, and finally detected by SYBR Green qPCR method for the titer of the recombinant adeno-associated virus. The titer of the finally obtained rAAV-CMVen-Dnah5 promoter-NLS-mNeonGreen-WPRE-SV40 virus was 1×1013 vg/mL.

### 3. In vivo verification of the Dnah5 promoter

The rAAV-CMVen-Dnah5 promoter-NLS-mNeonGreen-WPRE-SV40 virus was injected into the cochlea of a 2-3 day-old C57BL/6J mouse through the round window. After the virus was fully expressed for 10-14 days, the mouse was executed by cervical dislocation. The injected cochlea was removed and placed in 4% paraformaldehyde and fixed at room temperature for 2 hours, washed with PBS three times, for 5 min each time, then decalcified with 0.5 mM EDTA, and soaked at room temperature for 2 hours to soften the cochlea. The basilar membrane was dissected under a stereo microscope with an ophthalmic scalpel into three parts: the apex, middle, and base, and the vestibular utricle tissue. By means of immunofluorescence staining, hair cells were labeled with anti-Myo7a antibodies and supporting cells were labeled with anti-Sox2 antibodies to prepare samples. The samples were imaged with a laser confocal scanning microscope, showing that virus-infected cells would express green fluorescent protein in their nuclei. The wavelength of the excitation light was 488 nm, 561 nm, 647 nm, respectively. The in vivo assay results are shown in FIG. 23. This figure shows smear slide diagrams of vestibule (a) and cochlea (b) injected with rAAV-CMVen-Dnah5 promoter-NLS-mNeonGreen-WPRE-SV40 virus, in which purple indicates a marker protein Myo7a of hair cells, red indicates a marker protein Sox2 of supporting cells, green indicates virus-infected cells, with the results indicating that the Dnah5 specifically expresses fluorescent protein in vestibular hair cells.

The foregoing is merely a preferred embodiment of the present disclosure and should not be construed as a limitation of the present disclosure in any form or substance. It should be noted that, for a person of ordinary skill in the art, various improvements and supplements can be made without departing from the method of the present disclosure, which should also be regarded as falling within the scope of protection of the present disclosure. Any equivalent changes, modifications and evolutions made by those skilled in the art without departing from the spirit and scope of the present disclosure based on the above-disclosed technical contents are all equivalent embodiments of the present disclosure. At the same time, any equivalent changes, modifications and evolutions made to the above embodiments in accordance with the essential technology of the present disclosure are still within the scope of the technical solution of the present disclosure.

## Claims

1. A cochlear and/or vestibular cell-specific promoter, wherein,
a) the cell-specific promoter comprises nucleotide sequences shown as SEQ ID NOs: 1, 6, 11, 16, 21, 26, and 31; or,
b) the cell-specific promoter comprises nucleotide fragments having a sequence identity of more than 80% to SEQ ID NOs: 1, 6, 11, 16, 21, 26, 31 and having a function of the cell-specific promoter in a).

2. A primer pair for amplifying the cell-specific promoter of claim 1, comprising a forward primer and a reverse primer, wherein, the primer pair is selected from one of the following primer pairs A to G:
primer pair A: the nucleotide sequence of the forward primer is shown as SEQ ID NO: 3; the nucleotide sequence of the reverse primer is shown as SEQ ID NO: 4;
primer pair B: the nucleotide sequence of the forward primer is shown as SEQ ID NO: 8; the nucleotide sequence of the reverse primer is shown as SEQ ID NO: 9;
primer pair C: the nucleotide sequence of the forward primer is shown as SEQ ID NO: 13; the nucleotide sequence of the reverse primer is shown as SEQ ID NO: 14;
primer pair D: the nucleotide sequence of the forward primer is shown as SEQ ID NO: 18; the nucleotide sequence of the reverse primer is shown as SEQ ID NO: 19;
primer pair E: the nucleotide sequence of the forward primer is shown as SEQ ID NO: 23; the nucleotide sequence of the reverse primer is shown as SEQ ID NO: 24;
primer pair F: the nucleotide sequence of the forward primer is shown as SEQ ID NO: 28; the nucleotide sequence of the reverse primer is shown as SEQ ID NO: 29;
primer pair G: the nucleotide sequence of the forward primer is shown as SEQ ID NO: 33; the nucleotide sequence of the reverse primer is shown as SEQ ID NO: 34.

3. A cochlear and/or vestibular cell-specific recombinant vector comprising the cell-specific promoter of claim 1.

4. The recombinant vector of claim 3, wherein the recombinant vector is selected from one or more of an adeno-associated viral vector, an adenoviral vector, a lentiviral vector, a retroviral vector, a herpes simplex viral vector, a herpes simplex viral amplicon vector, a pseudorabies viral vector, a baculoviral vector, and a poxviral vector; preferably, an adeno-associated viral vector.

5. The recombinant vector of claim 4, wherein a gene for the cell-specific promoter is inserted at a Kpn1/Mlu1 or Xbal/BamHI multiple cloning site of a basic vector.

6. The recombinant vector of claim 5, wherein, the recombinant vector is selected from one of the following:
1) the specific promoter comprising a nucleotide sequence shown as SEQ ID NO: 1 is inserted at the Kpn1/Mlu1 multiple cloning site of the basic vector;
2) the specific promoter comprising a nucleotide sequence shown as SEQ ID NO: 6 is inserted at an Xbal/Nhel multiple cloning site of the basic vector;
3) the specific promoter comprising a nucleotide sequence shown as SEQ ID NO: 11 is inserted at the Xbal/Nhel multiple cloning site of the basic vector;
4) the specific promoter comprising a nucleotide sequence shown as SEQ ID NO: 16 is inserted at the Xbal/Nhel multiple cloning site of the basic vector;
5) the specific promoter comprising a nucleotide sequence shown as SEQ ID NO: 21 is inserted at the Xbal/Nhel multiple cloning site of the basic vector;
6) the specific promoter comprising a nucleotide sequence shown as SEQ ID NO: 26 is inserted at the Xbal/Nhel multiple cloning site of the basic vector;
7) the specific promoter comprising a nucleotide sequence shown as SEQ ID NO: 31 is inserted at the Kpn1/Mlu1 multiple cloning site of the basic vector.

7. The recombinant vector of claim 3, wherein, the recombinant vector further comprises a target gene; and/or, the target gene is located at the 3' end of the specific promoter gene.

8. The recombinant vector of claim 7, wherein, comprising at least any one of:
1) the target gene is selected from a human deafness-causing gene;
2) the target gene is expressed in the cochlear and/or vestibular cell;
3) the target gene is selected from Prestin, otof, vglut3, and Gjb2.

9. A pharmaceutical composition, comprising the recombinant vector of any one of claims 3 to 8.

10. A use of the cell-specific promoter of claim 1 or the primer pair of claim 2 or the recombinant vector of any one of claims 3 to 8 or the pharmaceutical composition of claim 9, wherein the use is selected from at least one of the following:
1) a use in preparing a medicine for preventing hearing-related diseases;
2) a use in preparing a medicine for gene therapies of hearing-related diseases;
3) a use in preparing and screening a medicine or vector for preventing and/or treating hearing-related diseases;
4) a use in constructing animal models of hearing-related diseases;
5) a use in constructing animal models of diseases accompanied with deafness-causing genetic abnormalities;
6) a use in in vitro studies related to the regeneration of cochlear outer hair cells, cochlear inner hair cells, cochlear supporting cells, vestibular hair cells, and cochlear type I and type II spiral ganglion neurons;
7) a use in in vitro studies related to hair cell damage protection;
8) a use in in vitro studies related to inner ear development;
9) a use in in vitro studies related to the pathogenesis of hearing-related diseases;
10) a use in in vitro studies related to auditory synaptic reconstruction.

11. A method for treating hearing-related diseases, comprising:
administering to a patient in need an effective amount of the recombinant vector of any one of claims 3 to 8.

12. The use of claim 10 or the method of claim 11, wherein the diseases are hearing-related diseases; preferably, the hearing-related diseases are selected from sensorineural hearing loss or tinnitus; further preferably, the sensorineural hearing loss is selected from a group of presbycusis, noise-induced hearing loss, ototoxic drug-associated hearing loss, sudden hearing loss, ischemic deafness, autoimmune deafness, Meniere's disease, head injury-associated hearing loss, hereditary hearing loss, dysfunction of the organ of Corti due to other or unknown causes, or damage to organ of Corti caused by viral or bacterial infection, or a combination thereof.
